# EUROPEAN PATENT SPECIFICATION

(11) **EP 3 746 469 B1**
(45) Date of publication and mention of the grant of the patent: **10.07.2024**
(21) Application number: 19702096.9
(22) Date of filing: 01.02.2019
(51) Int. Cl.: C07K 14/81, A61K 38/57, C07K 14/435, A61P 7/02

(54) **ANTICOAGULANT PROTEINS AND THEIR USE FOR TREATING DISEASES ASSOCIATED WITH THE ACTIVATION OF NEUTROPHILS**
ANTIKOAGULIERENDE PROTEINE UND DEREN VERWENDUNG ZUR BEHANDLUNG VON ERKRANKUNGEN IM ZUSAMMENHANG MIT DER AKTIVIERUNG VON NEUTROPHILEN
PROTÉINES ANTICOAGULANTES ET LEUR UTILISATION POUR TRAITER DES MALADIES ASSOCIÉES À L'ACTIVATION DE NEUTROPHILES

(30) Priority: 01.02.2018 US 201862624997 P; 27.07.2018 EP 18186061
(43) Date of publication of application: 09.12.2020
(60) Divisional application: 24175271.6
(73) Proprietor: Bioxodes, 6041 Gosselies (BE)
(72) Inventor: PIREAUX, Valérie, 5000 Namur (BE); DEMOULIN, Stéphanie, 4520 Antheit (BE); GODFROID, Edmond, 1120 Bruxelles (BE); TASSIGNON, Joël, 6044 Roux (BE); DEROCHETTE, Sandrine, 4520 Huccorgne (BE); GUYAUX, Michel, 1150 Woluwe-Saint-Pierre (BE)
(74) Representative: Icosa Europe
(86) International application number: PCT/EP2019/052542
(87) International publication number: WO 2019/149906

(56) References cited:
- EP-A2- 1 892 297
- WO-A1-2009/141382
- US-A1- 2016 046 728
- GOEBEL SILVIA ET AL: "The GPVI-Fc Fusion Protein Revacept Improves Cerebral Infarct Volume and Functional Outcome in Stroke", PLOS ONE, vol. 8, no. 7, 23 July 2013 (2013-07-23), pages e66960, XP055925881, Retrieved from the Internet <URL:https://www.ncbi.nlm.nih.gov/pmc/articles/PMC3720811/pdf/pone.0066960.pdf> DOI: 10.1371/journal.pone.0066960
- DARBOUSSET R: "Ir-CPI: An original inhibitor of thrombosis and thrombosis-associated with cancer", RESEARCH AND PRACTICE IN THROMBOSIS AND HAEMOSTASIS 20180701 WILEY-BLACKWELL PUBLISHING LTD NLD, vol. 2, no. Supplement 1, 1 July 2018 (2018-07-01), pages 244 - 245, XP009508686, ISSN: 2475-0379
- YVES DECREM ET AL: "Ir-CPI, a coagulation contact phase inhibitor from the tick Ixodes ricinus , inhibits thrombus formation without impairing hemostasis", THE JOURNAL OF EXPERIMENTAL MEDICINE, vol. 206, no. 11, 5 October 2009 (2009-10-05), US, pages 2381 - 2395, XP055496196, ISSN: 0022-1007, DOI: 10.1084/jem.20091007
- DATABASE Geneseq [online] 7 April 2016 (2016-04-07), "Ixodes ricinus Ir-CPI protein SEQ ID NO: 36.", XP002785799, retrieved from EBI accession no. GSP:BCM20403 Database accession no. BCM20403
- JIANG PENGFEI ET AL: "The extrinsic coagulation cascade and tissue factor pathway inhibitor in macrophages: A potential therapeutic opportunity for atherosclerotic thrombosis", THROMBOSIS RESEARCH, vol. 133, no. 4, 15 January 2014 (2014-01-15), pages 657 - 666, XP028638209, ISSN: 0049-3848, DOI: 10.1016/J.THROMRES.2014.01.012
- EZZAT WALEED H ET AL: "Recombinant human tissue factor pathway inhibitor prevents thrombosis in a venous tuck model.", THE LARYNGOSCOPE NOV 2010, vol. 120, no. 11, November 2010 (2010-11-01), pages 2172 - 2176, XP002785800, ISSN: 1531-4995
- R. DARBOUSSET ET AL: "Tissue factor-positive neutrophils bind to injured endothelial wall and initiate thrombus formation", BLOOD, vol. 120, no. 10, 26 July 2012 (2012-07-26), US, pages 2133 - 2143, XP055515557, ISSN: 0006-4971, DOI: 10.1182/blood-2012-06-437772

## Description

### FIELD OF INVENTION

The present invention relates to proteins and polypeptides comprising an *Ixodes ricinus* salivary gland polypeptide, and their use for treating and/or preventing diseases and conditions associated with the activation of neutrophils.

### BACKGROUND OF INVENTION

Hemostasis is a vital function that stops bleeding and protects the integrity of blood circulation on both molecular and macroscopic levels. Hemostasis includes a coagulation cascade traditionally divided into two converting enzymatic cascades that are triggered either by blood-borne components of the vascular system (the intrinsic pathway) or by exposure of blood to a damaged vessel wall (the extrinsic pathway).

The intrinsic pathway and the contact phase (also referred to as the plasma kallikreinkinin system), is initiated by contact phase proteins including the zymogens Factor XII (FXII), Factor XI (FXI) and prekallikrein (pKK or PK), as well as the cofactor high molecular weight kininogen (HK). Factor XII undergoes autoactivation when bound to negatively charged surfaces, generating activated Factor XII (FXIIa or αFXIIa) by a conformation change. αFXIIa then converts PK into activated plasma kallikrein (KK or PKa). Once small amounts of PKa are formed, they catalyze the conversion of surface-bound Factor XII into αFXIIa leading to strong positive feedback on the system. During this process, the activation of Factor XII leads to a succession of proteolysis steps leading to the production of a series of different active enzymes (XIa, IXa, VIIIa, Xa) which ultimately leads to activation of pro-thrombin in thrombin and formation of fibrin.

The extrinsic coagulation pathway, or Tissue Factor (TF) pathway, consists of a number of serine proteases, cofactors, calcium, and cell membrane components. The extrinsic coagulation pathway is activated by TF, also called platelet Tissue Factor, Factor III, thromboplastin, or CD142, which is expressed in the subendothelial layer. The extrinsic coagulation pathway is thus triggered by the binding of TF produced by exposed subendothelial cells to plasma Factor VII. The resulting complex initiates the coagulation cascade, converts Factor X into Factor Xa which ultimately leads to thrombin generation. Thrombin activates platelets and converts fibrinogen into fibrin. Both platelets and fibrin are essential elements of the hemostatic plug that is responsible for sealing the vascular breach. The extrinsic pathway is proposed to be the primary activator of the coagulation protease cascade *in vivo.* Subsequently, propagation of the thrombus involves recruitment of additional platelets and amplification of the coagulation cascade.

Moreover, under pathologic conditions such as inflammatory stimuli, TF is expressed by monocytes, neutrophils, endothelial cells, and platelets, which results in an elevation of the levels of circulating TF-positive microparticles. Therefore, the extrinsic coagulation pathway, besides activation of coagulation factors, also involves cells. Importantly, platelets play a critical role in the amplification of the coagulation cascade by providing a thrombogenic surface.

Studies conducted over the last decade support the growing notion that neutrophils contribute significantly to the thrombotic process. The attenuation of thrombotic manifestations in thrombotic animal models depleted for neutrophils demonstrates the contribution of these cells in thrombosis. Neutrophils can contribute to the pathologic venous and arterial thrombosis by the release of neutrophil extracellular traps (NETs). NET release is emerging as a major contributor to thrombogenesis in pathologic conditions such as sepsis, deep vein thrombosis and malignancy. In addition, it is suggested that NETs provide the scaffold for fibrin deposition and platelet entrapment and subsequent activation. Moreover, blood-cell derived microparticles, including those from neutrophils, have been involved in thrombus formation. Several studies also support the *in vivo* and *ex vivo* TF production by neutrophils (Kambas et al., 2012).

The active role of neutrophils in *in vivo* experimental thrombosis and inflammation-driven thrombotic diseases has been demonstrated. A contribution of neutrophils in the activation of the extrinsic coagulation cascade is the degradation of TFPI via elastase release, TFPI being the main inhibitor of TF (Massberg et al., 2010). In addition, it was demonstrated that neutrophil binding to the injured endothelium was the initial step in the continuum of events that results in thrombus formation in a model of laser-induced endothelial injury. The critical role of neutrophils was reinforced by the observation that these cells were the main source of TF, which was required for thrombus formation. It was shown that inhibition of neutrophil binding to the vessel wall reduces the presence of TF and diminishes the generation of fibrin and platelet accumulation (Darbousset et al., 2012; Darbousset et al., 2014). Moreover, in the same model, Factor XII deficiency did not attenuate thrombus generation (Darbousset et al., 2012). Thus, neutrophils play an important role in the activation of extrinsic coagulation system and NETs could provide the scaffold for fibrin deposition and platelet entrapment and subsequent activation.

For the past fifty years, anticoagulant treatment has been dominated by two classes of agents: heparins and antivitamins K. Heparins accelerate the inhibitory action of antithrombin on some activated coagulation factors (specifically thrombin and Factors IXa and Xa) by indirect inhibition of these factors and are only active when administered parenterally. Antivitamins K prevent the final synthesis of four coagulation factors (prothrombin and Factors VII, IX and X). Both classes of agents inhibit the extrinsic pathway of coagulation. However, the therapeutic window of these agents is narrow, requiring careful monitoring of patients. Indeed, these agents require careful laboratory testing in order to guarantee sufficient antithrombotic effectiveness while avoiding the risk of hemorrhage.

Thus, there is a strong need for new anticoagulants with no hemorrhagic side effects.

The Applicant previously demonstrated that polypeptides isolated from the salivary gland of the tick *I. ricinus,* Ir-CPI (*Ixodes ricinus* Contact Phase Inhibitor), specifically target coagulation Factors XIa and XIIa (EP1892297 and EP2123670; Decrem et al., 2009). *Ixodes ricinus* salivary gland polypeptides were thus found to be specific inhibitors of the intrinsic coagulation pathway. Indeed, *in vitro,* Ir-CPI was reported to prolong the activated Partial Thromboplastin Time (aPTT), showing interference with the intrinsic pathway, while having no effect on the Prothrombin Time (PT), the Thrombin Time (TT), and the dilute Russell's Viper Venom time (dRVVT), three tests triggering blood coagulation with activators of the extrinsic or common pathway. Moreover, in contrast to its strong dose-dependent reduction of thrombin generation induced by ellagic acid (intrinsic pathway activator), Ir-CPI was reported to be quite inactive in a thrombin generation assay in which thrombin generation was induced by low concentration of TF (5 pM) (extrinsic pathway activator). The small inhibition of thrombin generation with 5 pM of TF is explained by the fact that, at low concentrations of TF, thrombin can activate FXI of the intrinsic pathway in order to create a feedback-loop involving thrombin, FXI(a), FIX(a), FX(a) and (pro)thrombin, that sustains the extrinsic pathway (Keularts, Zivelin et al. 2001).

As a potential mechanism of action, the molecule was shown to inhibit activation of Factor XI and PK by Factor XIIa and of Factor XII by Factor XIa but was totally inactive on other targets of the coagulation pathways especially thrombin and Factor Xa which are the targets of the currently available parenteral and/or oral anticoagulant and antithrombotic drugs. Results previously disclosed were obtained in *in vitro* assays only involving factors of the coagulation pathways and, therefore, could not suggest an activity on cells involved in the extrinsic coagulation pathway.

Because of its specific effect on the intrinsic coagulation pathway, Ir-CPI is expected to have a larger therapeutic window regarding the risk of bleeding which constitutes the main side effect of current parenteral anticoagulants. As a matter of fact, the Applicant demonstrated that the molecule does not increase bleeding in an experimental model in rodents at pharmacological therapeutic active dose (Decrem, Rath et al., 2009).

The Applicant has now found that *Ixodes ricinus* salivary gland polypeptides unexpectedly inhibit the recruitment of polymorphonuclear neutrophils (PMNs) and platelets at the sites of lesion in an experimental mouse arteriolar laser injury model reported to be strictly dependent of TF and independent of FXII (Darbousset et al., 2012). In addition, the Applicant also found that, *in vitro, Ixodes ricinus* salivary gland polypeptides inhibit the activation of PMNs and the formation of neutrophil extracellular traps (also called NETosis). While not willing to be bound by any theory, the Applicant thus concludes that Ir-CPI inhibits the recruitment and activation of neutrophils at the site of lesion and thus plays a role in the recruitment and activation of cells involved when the extrinsic coagulation pathway is activated.

Therefore, *Ixodes ricinus* salivary gland polypeptides are useful for the treatment and/or the prevention of diseases and conditions associated with the activation of the extrinsic coagulation pathway, such as, for example, thrombosis, and for the treatment and/or the prevention of inflammatory diseases with thrombotic tendency and thromboinflammation.

The discovery of these new features in the mechanism of action of Ir-CPI polypeptides makes of these molecule original compounds not only capable of inhibiting the intrinsic pathway but also capable to act on coagulation and/or thrombotic process involving TF but via a totally different mechanism that the inhibitors of thrombin and/or of Factors Xa.

The present invention thus relates to anticoagulant proteins comprising an *Ixodes ricinus* salivary gland polypeptide, and their use for treating and/or preventing diseases and conditions associated with the activation of neutrophils and/or NETosis.

### SUMMARY

The present invention relates to a protein or polypeptide comprising a polypeptide that has at least 85 % sequence identity with the amino acid sequence SEQ ID NO: 1 for use for treating and/or preventing thromboinflammation.

In one embodiment, said thromboinflammation is selected from the group comprising atherosclerosis, plaque rupture, devices-induced thromboinflammation, thrombosis induced by catheterism and/or stent positioning procedures, thrombosis induced by extracorporeal circulation, thrombus formation following cerebral injuries, coronary artery disease, acute myocardial infraction, cancer-associated thrombosis, metastasis-associated thrombosis, stroke-associated thrombosis, Behçet's disease (BD), antineutrophil cytoplasmic antibody-associated (ANCA) vasculitides, Takayasu arteritis, rheumatoid arthritis, systemic lupus erythematosus, antiphospholipid syndrome, familial Mediterranean fever, thromboangiitis obliterans (TAO), sepsis, inflammatory bowel diseases, heparin-induced thrombocytopenia, immunothrombosis, thrombosis associated with preeclampsia, thrombotic complication in cell and cell cluster transplantation and in whole organ transplantation or grafts, venous thromboembolism, aneurysms, and skeletal muscle ischemia-reperfusion syndrome.

In one embodiment, said thromboinflammation is thrombosis induced by catheterism and/or stent positioning procedures at the site of local vascular stenosis. In another embodiment, said thromboinflammation is blood-contacting medical devices-induced thromboinflammation. In another embodiment, said thromboinflammation is thrombosis and/or coagulation associated to the extracorporeal circulation.

The invention also relates to a pharmaceutical composition comprising a protein or polypeptide comprising a polypeptide that has at least 85 % sequence identity with the amino acid sequence SEQ ID NO: 1 and at least one pharmaceutically acceptable excipient for use for treating and/or preventing thromboinflammation.

The invention also relates to a medicament comprising a protein or polypeptide comprising a polypeptide that has at least 85 % sequence identity with the amino acid sequence SEQ ID NO: 1 for use for treating and/or preventing thromboinflammation.

Another object of the present invention is a medical device coated with an isolated polypeptide that has at least 85 % sequence identity with the amino acid sequence SEQ ID NO: 1 for use for treating and/or preventing thromboinflammation.

Still another object of the present invention is a kit comprising a protein or polypeptide comprising an isolated polypeptide that has at least 85 % sequence identity with the amino acid sequence SEQ ID NO: 1 for use for treating and/or preventing thromboinflammation.

### DEFINITIONS

In the present invention, the following terms have the following meanings:
The term **"about"** preceding a value means plus or minus 10% of said value.

The term **"amino acid substitution"** refers to the replacement in a polypeptide of one amino acid with another amino acid. In one embodiment, an amino acid is replaced with another amino acid having similar structural and/or chemical properties, *e.g.,* conservative amino acid replacements. "Conservative amino acid substitution" may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; negatively charged (acidic) amino acids include aspartic acid and glutamic acid. Non-conservative substitutions will entail exchanging a member of one of these classes for another class. For example, amino acid substitutions can also result in replacing one amino acid with another amino acid having different structural and/or chemical properties, for example, replacing an amino acid from one group (*e.g.,* polar) with another amino acid from a different group (*e.g.,* basic). Amino acid substitutions can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful.

The term **"identity"** refers to a measure of the identity of nucleotide sequences or amino acid sequences. In general, the sequences are aligned so that the highest order match is obtained. "Identity" per se has an art-recognized meaning and can be calculated using published techniques. See, *e.g*.: Computational Molecular Biology, Lesk, A.M., ed., Oxford University Press, New York, 1988; Biocomputing: Informatics And Genome Projects, Smith, D.W., ed., Academic Press, New York, 1993; Computer Analysis Of Sequence Data, Part I, Griffin, A.M., and Griffin, H.G., eds, Humana Press, New Jersey, 1994; Sequence Analysis In Molecular Biology, von Heijne, G., Academic Press, 1987; and Sequence Analysis Primer, Gribskov, M. and Devereux, J., eds, M Stockton Press, New York, 1991. While there exist a number of methods to measure identity between two polynucleotide or polypeptide sequences, the term "identity" is well known to skilled artisans (Carillo and Lipton, SIAM J Applied Math, 1998, 48:1073). Methods commonly employed to determine identity or similarity between two sequences include, but are not limited to, those disclosed in Guide to Huge Computers, Martin J. Bishop, ed., Academic Press, San Diego, 1994; and Carillo and Lipton, SIAM J Applied Math, 1998, 48:1073. Methods to determine identity and similarity are codified in computer programs. Preferred computer program methods to determine identity and similarity between two sequences include, but are not limited to, GCG program package (Devereux et al., J Molec Biol, 1990, 215:403). Most preferably, the program used to determine identity levels was the GAP program, as was used in the Examples below.

As an illustration, by a polynucleotide having a nucleotide sequence having at least, for example, 95% "identity" to a reference nucleotide sequence is intended that the nucleotide sequence of the polynucleotide is identical to the reference sequence except that the polynucleotide sequence may include an average up to five point-mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

The term **"peptide linker",** also called "spacer peptide", refers to a peptide used to link 2 peptides or polypeptides together. In one embodiment, a peptide linker of the invention comprises from 3 to 50 amino acids. Peptide linkers are known in the art or are described herein.

The term **"pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like.

A pharmaceutically acceptable carrier or excipient refers to a non-toxic solid, semi-solid or liquid filler, diluent, encapsulating material or formulation auxiliary of any type. For human administration, preparations should meet sterility, pyrogenicity, general safety and purity standards as required by FDA Office of Biologics standards.

The term **"polynucleotide"** refers to any polyribonucleotide or polydeoxyribonucleotide, which may be unmodified RNA or DNA or modified RNA or DNA. "Polynucleotides" include, without limitation single-and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions. In addition, "Polynucleotide" refers to triple-stranded regions comprising RNA or DNA or both RNA and DNA. The term Polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "Polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

The term **"polypeptide"** refers to any peptide or protein comprising two or more amino acids joined to each other by peptide bonds or modified peptide bonds, *i.e.,* peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids.

The term **"protein"** refers to a sequence of more than 100 amino acids and/or to a multimeric entity. The proteins of the invention are not limited to a specific length of the product. The term "polypeptide" or "protein" does not refer to or exclude post-expression modifications of the protein, for example, glycosylation, acetylation, phosphorylation and the like, as well as other modifications known in the art, both naturally occurring and non-naturally occurring. Such modifications are well described in basic texts and in more detailed monographs, as well as in a voluminous research literature. Modifications can occur anywhere in a polypeptide or protein, including the peptide backbone, the amino acid side-chains and the amino or carboxyl termini. It will be appreciated that the same type of modification may be present in the same or varying degrees at several sites in a given polypeptide or protein. Also, a given polypeptide or protein may contain many types of modifications. Polypeptides or proteins may be branched as a result of ubiquitination, and they may be cyclic, with or without branching. Cyclic, branched and branched cyclic polypeptides or proteins may result from posttranslational natural processes or may be made by synthetic methods. Modifications include acetylation, acylation, ADP-ribosylation, amidation, covalent attachment of flavin, covalent attachment of a hem moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-linkings, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino of amino acids to proteins such as arginylation, and ubiquitination. See, for instance, "Proteins-structure and molecular properties", 2nd Ed., T. E. Creighton, W. H. Freeman and Comany, New York, 1993; Wolt, F., "Posttranslational Protein Modifications: Perspectives and Prospects", Posttranslational covalent modification of proteins, B. C. Johnson, Ed., Academic Press, New York, 1983, pgs. 1-12; Seifter et al., "Analysis for protein modifications and nonprotein cofactors", Meth Enzymol, 1990, 182:626-646; Rattan et al, "Protein Synthesis: Posttranslational Modifications and Aging", Ann NY Acad Sci, 1992, 663:48-62. A protein may be an entire protein, or a subsequence thereof.

An **"isolated protein or polypeptide"** is one that has been identified and separated and/or recovered from a component of its natural environment. In a preferred embodiment, the isolated protein or polypeptide will be purified
(1) to greater than 80, 85, 90, 95% by weight of protein or polypeptide as determined by the Lowry method, and most preferably more than 96, 97, 98, or 99% by weight,
(2) to a degree sufficient to obtain at least 15 residues of N-terminal or internal amino acid sequence by use of a spinning cup sequenator, or
(3) to homogeneity by SDS-PAGE under reducing or non-reducing conditions using Coomassie blue or, preferably, silver staining.

Isolated protein or polypeptide includes the protein *in situ* within recombinant cells since at least one component of the protein's or polypeptide's natural environment will not be present. Ordinarily, however, isolated protein or polypeptide will be prepared by at least one purification step.

The term **"fusion protein"** refers to a molecule comprising two or more proteins or fragments thereof linked by a covalent bond via their individual peptide backbones, most preferably generated through genetic expression of a polynucleotide molecule encoding those proteins. The polypeptides forming the fusion protein are typically linked C-terminus to N-terminus, although they can also be linked C-terminus to C-terminus, N-terminus to N-terminus, or N-terminus to C-terminus. The polypeptides of the fusion proteins may be fused in any order.

The term **"fused"** refers to components that are linked by peptide bonds, either directly or through one or more peptide linkers.

The term **"native Ir-CPI"** refers to naturally occurring Ir-CPI, as opposed to a **"modified Ir-CPI",** which has been modified from a naturally occurring Ir-CPI, *e.g.,* to alter one or more of its properties such as stability. A modified Ir-CPI polypeptide may for example comprise modifications in the amino acid sequence, *e.g*., amino acid substitutions, deletions or insertions.

The term **"subject"** refers to a mammal, preferably a human. In one embodiment, the subject is a man. In another embodiment, the subject is a woman. In one embodiment, a subject may be a "patient", *i.e.,* a warm-blooded animal, more preferably a human, who/which is awaiting the receipt of, or is receiving medical care or was/is/will be the object of a medical procedure, or is monitored for the development of the disease or condition. In one embodiment, the subject is an adult (for example a subject above the age of 18). In another embodiment, the subject is a child (for example a subject below the age of 18).

The term **"therapeutically effective amount"** means the level or amount of agent that is aimed at, without causing significant negative or adverse side effects to the target, (1) delaying or preventing disease or condition associated to the recruitment of neutrophils, the activation of neutrophils or to the activation of the extrinsic coagulation pathway; (2) slowing down or stopping the progression, aggravation, or deterioration of one or more symptoms of disease or condition associated to the recruitment of neutrophils, the activation of neutrophils or to the activation of the extrinsic coagulation pathway; (3) bringing about ameliorations of the symptoms of disease or condition associated to the recruitment of neutrophils, the activation of neutrophils or to the activation of the extrinsic coagulation pathway; (4) reducing the severity or incidence of disease or condition associated to the recruitment of neutrophils, the activation of neutrophils or to the activation of the extrinsic coagulation pathway; or (5) preventing disease or condition associated to the recruitment of neutrophils, the activation of neutrophils or to the activation of the extrinsic coagulation pathway. In one embodiment, a therapeutically effective amount is administered prior to the onset of disease or condition associated to the recruitment of neutrophils, the activation of neutrophils or to the activation of the extrinsic coagulation pathway, for a prophylactic or preventive action.

The term **"treating"** or **"treatment"** or **"alleviation"** refers to both therapeutic treatment and prophylactic or preventative measures; wherein the object is to prevent or slow down (lessen) the disease or condition associated to the recruitment of neutrophils, the activation of neutrophils or to the extrinsic coagulation pathway. Those in need of treatment include those already affected with the disease or condition associated to the recruitment of neutrophils, the activation of neutrophils or to the extrinsic coagulation pathway as well as those prone to have the disease or condition associated to the recruitment of neutrophils, the activation of neutrophils or to the extrinsic coagulation pathway or those in whom the disease or condition associated to the recruitment of neutrophils, the activation of neutrophils or to the extrinsic coagulation pathway is to be prevented. A subject or mammal is successfully "treated" for an infection if, after receiving a therapeutic amount of a protein or polypeptide according to the methods of the present invention, the patient shows observable and/or measurable reduction in or absence of one or more of the following: reduction in the number of pathogenic cells; reduction in the percent of total cells that are pathogenic; and/or relief to some extent, one or more of the symptoms associated with the disease or condition associated to the recruitment of neutrophils, the activation of neutrophils or to the extrinsic coagulation pathway; reduced morbidity and mortality, and improvement in quality of life issues. The above parameters for assessing successful treatment and improvement in the disease or condition associated to the recruitment of neutrophils, the activation of neutrophils or to the extrinsic coagulation pathway are readily measurable by routine procedures familiar to a physician.

The term **"variant"** refers to a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions identical. A variant and reference polypeptide may differ in amino acid sequence by one or more substitutions (preferably conservative), additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. Variants should retain one or more of the biological activities of the reference polypeptide.

### DETAILED DESCRIPTION

The present invention thus relates to a protein or polypeptide comprising an *Ixodes ricinus* salivary gland polypeptide or a fragment or a variant thereof, and its use for inhibiting the extrinsic coagulation pathway and thereby for treating and/or preventing diseases and conditions associated to the extrinsic coagulation pathway, in particular treating and/or preventing thrombus associated to the activation of the extrinsic coagulation pathway. In one embodiment, the protein or polypeptide of the invention is an isolated protein or polypeptide.

In one embodiment, the *Ixodes ricinus* salivary gland polypeptide of the invention is Ir-CPI (*Ixodes ricinus* Contact Phase Inhibitor). As used herein, Ir-CPI may also be called IrCPI.

According to one embodiment, the Ir-CPI polypeptide of the invention has an amino sequence comprising or consisting in the sequence SEQ ID NO: 1. In one embodiment, the Ir-CPI polypeptide of the invention is encoded by the cDNA corresponding to the amino acid sequence SEQ ID NO: 1.

In one embodiment, the amino acid sequence of the Ir-CPI polypeptide of the invention is at least 85% identical to SEQ ID NO: 1, preferably at least 90%, 95%, 96%, 97%, 98%, 99% or 100% identical.

As used herein, the term "fragment" means a polypeptide having an amino acid sequence that is the same as part, but not all, of the amino acid sequence of the aforementioned Ir-CPI polypeptide. As with the Ir-CPI polypeptide, fragments may be "free-standing" or comprised within a larger polypeptide of which they form a part or region, most preferably as a single continuous region. Representative examples of polypeptide fragments include, for example, fragments from about amino acid number 1-20, 21-40, 41-60, 61-80, 81-100, and 101 to the end of the polypeptide. In this context "about" includes the particularly recited ranges larger or smaller by several, 5, 4, 3, 2 or 1 amino acid at either extreme or at both extremes. Preferred fragments include, but are not limited to, truncation polypeptides having the amino acid sequence of the Ir-CPI polypeptide, except for deletion of a continuous series of residues that includes the amino terminus, or a continuous series of residues that includes the carboxyl terminus and/or transmembrane region or deletion of two continuous series of residues, one including the amino terminus and one including the carboxyl terminus. Also preferred are the fragments characterized by structural or functional attributes such as fragments that comprise alpha-helix and alpha-helix forming regions, beta-sheet and beta-sheet forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate Ir-CPI polypeptide activity, including those with a similar activity or an improved activity, or with a decreased undesirable activity.

All fragments retain parts of the biological activity of the Ir-CPI polypeptide.

Preferred variants are those that vary from the Ir-CPI polypeptide by conservative amino acid substitutions, *i*.*e*., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination. Most preferred variants are naturally occurring allelic variants of the Ir-CPI polypeptide present in *Ixodes ricinus* salivary glands.

In one embodiment, the Ir-CPI polypeptide of the invention has an amino sequence comprising a mutation leading to the replacement of Asparagine in a position corresponding to position 54 in the amino acid sequence of SEQ ID NO: 1 by Glutamine in order to prevent N-glycosylation. In one embodiment, the Ir-CPI polypeptide of the invention has an amino sequence comprising or consisting in the sequence of SEQ ID NO: 2.

The Ir-CPI polypeptide of the invention can be prepared in any suitable manner. Example of peptides prepared according to a suitable manner include, without being limited to, isolated naturally occurring polypeptides, recombinantly produced polypeptides, synthetically produced polypeptides, or polypeptides produced by a combination of these methods. Means for preparing such polypeptides are well understood in the art.

In one embodiment, the protein of the invention is a fusion protein comprising Ir-CPI. As used herein, the term "fusion protein" refers to a fusion of the Ir-CPI polypeptide as described hereinabove and at least one other polypeptide.

In one embodiment, the polypeptides of the fusion protein are linked C-terminus to N-terminus. In another embodiment, the components of the fusion protein are linked C-terminus to C-terminus. In another embodiment, the components of the fusion protein are linked N-terminus to N-terminus. In another embodiment, the components of the fusion protein are linked N-terminus to C-terminus.

In one embodiment, the polypeptides of the fusion protein may be fused in any order.

In one embodiment, the polypeptides are disposed in a single, contiguous polypeptide chain.

In one embodiment, the term "Ir-CPI-fusion proteins" or "fusion proteins" indiscriminately refers to fusion proteins without a linker sequence added or with a linker sequence added.

The linker peptide provides a greater physical separation between the two moieties and thus maximizes the availability of the *Ixodes ricinus* salivary gland polypeptide. The linker peptide may consist of amino acids such that it is flexible or more rigid.

It comprises from 1 to 60 amino acids, preferably from 2 to 50 amino acids, more preferably from 4 to 40 amino acids. In one embodiment, the peptide linker of the invention comprises from 5 to 50 amino acids, from 10 to 50 amino acids, from 15 to 50 amino acids, or from 20 to 50 amino acids. In another embodiment, the peptide linker of the invention comprises from 6 to 20 amino acids, from 8 to 20 amino acids, or from 10 to 20 amino acids. In another embodiment, the peptide linker of the invention comprises from 2 to 10 amino acids, from 5 to 20 amino acids, from 10 to 30 amino acids, or from 15 to 40 amino acids.

Fusion proteins of the invention may be obtained, for example, by solid-state peptide synthesis (*e.g.*, Merrifield solid phase synthesis) or recombinant production. For recombinant production one or more polynucleotide encoding the fusion protein (fragment), *e.g.,* as described hereinabove, is isolated and inserted into one or more vectors for further cloning and/or expression in a host cell. Such polynucleotide may be readily isolated and sequenced using conventional procedures.

Methods which are well known to those skilled in the art can be used to construct expression vectors containing the coding sequence of a fusion protein (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the fusion protein (fragment) (*i.e.,* the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, *e.g*., on a single vector, or in separate polynucleotide constructs, *e.g.,* on separate (different) vectors. Furthermore, any vector may contain a single coding region, or may comprise two or more coding regions, *e.g.,* a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the fusion protein (fragment) of the invention, or variant or derivative thereof. Heterologous coding regions include without limitation specialized elements or motifs, such as a secretory signal peptide or a heterologous functional domain. An operable association is when a coding region for a gene product, *e.g.,* a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (*e.g.*, the immediate early promoter, in conjunction with intron-A), simian virus 40 (*e.g.,* the early promoter), and retroviruses (such as, *e.g.,* Rous sarcoma virus). Other transcription control regions include those derived from vertebrate genes such as actin, heat shock protein, bovine growth hormone and rabbit a-globin, as well as other sequences capable of controlling gene expression in eukaryotic cells. Additional suitable transcription control regions include tissue-specific promoters and enhancers as well as inducible promoters (*e.g*., promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Fusion proteins prepared as described herein may be purified by art-known techniques such as high-performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the fusion protein binds. The purity of the fusion protein can be determined by any of a variety of well-known analytical methods including gel electrophoresis, high pressure liquid chromatography, and the like.

The present invention further relates to a pharmaceutical composition comprising a protein or polypeptide of the invention, and at least one pharmaceutically acceptable excipient.

Pharmaceutically acceptable excipients that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

Another object of the invention is a medicament comprising a protein or polypeptide or a pharmaceutical composition as described hereinabove.

In one embodiment, the pharmaceutical composition or the medicament of the invention comprises a therapeutically effective amount of the protein or polypeptide as described hereinabove.

A further object of the present invention is a medical device coated by a protein or polypeptide as described hereinabove.

Furthermore, another object of the present invention is the use of a protein or polypeptide as described hereinabove for coating a medical device. Still another object of the present invention is a protein or polypeptide as described hereinabove for coating a medical device.

Accordingly, the present invention also relates to a medical device coated with a protein or polypeptide as described hereinabove.

Examples of medical devices that may be coated by the protein or polypeptide of the invention include, but are not limited to, coronary stent, artificial heart, artificial heart valve, central venous line, cardioplegia delivery system, dilators, tunneling devices, stent grafts cannulae, catheters, extracorporeal circulation systems including but not limited to extracorporeal membrane oxygenation systems, (auto)transfusion systems, arterial filters, hemodialysis systems, plasmapheresis systems; medical device used for collection of blood outside the body; and/or accessories for any one of said devices including but not limited to tubing, cannulae, centrifugal pump, valve, port, and/or diverter, arterial stents for stenosis and aneurysm, and left ventricular assist devices.

The Applicant has surprisingly found that an Ir-CPI polypeptide according to the invention inhibits platelet recruitment, neutrophil recruitment, neutrophil activation and NETosis, thereby inhibiting thrombosis associated with the extrinsic coagulation pathway.

Indeed, the Applicant has shown that an Ir-CPI polypeptide revealed to be surprisingly protective in an experimental animal model of laser injury of the cremaster muscle arterioles wherein the extrinsic coagulation pathway is known to be the key factor leading to the thrombotic event (Darbousset et al., 2012). In this model, Ir-CPI inhibits not only the production of fibrin but also the recruitment of platelets at the site of the lesion. The Ir-CPI polypeptide was also active when the laser injury was applied on tumor-bearing mice in a model that is mainly dependent on Tissue Factor expressed by cancer cell-derived microparticles (Thomas et al., 2009). Since neutrophils play a key role in the activation of the Tissue Factor dependent pathway in the laser-injury model (Darbousset et al., 2012), the presence and activation of neutrophils were next compared in presence or absence of Ir-CPI. The Applicant observed that *in vivo,* Ir-CPI significantly inhibited the quantity of neutrophils accumulating and the neutrophil elastase activity at the site of injury. The Applicant also showed that *in vitro,* Ir-CPI significantly inhibited the activation of neutrophils primed with TNFα (tumor necrosis factor alpha) or incubated with Adenosine Triphosphate (ATP), as observed through the detection of the CD11b, CD11b activated or Ly6G expression at the surface of neutrophils. Upon activation, neutrophils may produce "neutrophil extracellular traps" (NETs), which are structures of chromatin filaments coated with histones, proteases and granular and cytosolic proteins. Studies suggest that NETs contribute to thrombosis by promoting fibrin deposition and platelet aggregation. The Applicant observed that *in vitro,* Ir-CPI also significantly diminished the formation of NETs (*i.e.,* NETosis) by neutrophils primed with TNFα and activated with PAF (platelet-activating factor). The contribution of neutrophils in the generation of thrombotic events has been demonstrated in various experimental paradigms. The implication of autophagy in NETs release and Tissue Factor delivery to NETs and the link between NETs and thrombosis suggests a critical role for neutrophils in the interaction between inflammatory and thrombotic pathways (Demers et al., 2012; Leal et al., 2017; Mauracher et al., 2018). The attenuation of thrombotic manifestations in thrombotic animal models by neutrophil depletion demonstrates the contribution of these cells in thrombosis (von Brühl et al., 2012). The expression of produced and/or acquired TF by neutrophils suggests an involvement of these neutrophils in the pathogenesis of thrombotic events that characterize several inflammatory disorders. Thus, Ir-CPI inhibits thrombus formation following activation of the extrinsic coagulation pathway not through the inhibition of coagulation factors specific of this pathway, such as Tissue Factor, but through the inhibition of platelet recruitment, neutrophil recruitment, neutrophil activation and/or NET formation.

The Applicant has thus surprisingly found that an Ir-CPI polypeptide can be used in therapeutic indications aimed at preventing and/or treating thrombotic events in diseases and conditions wherein thrombosis and/or coagulation depends on the platelet recruitment, neutrophil recruitment, neutrophil activation and/or NET formation. The Applicant also demonstrated that Ir-CPI can be used in therapeutic indications aimed at preventing and/or treating thrombotic events in diseases and conditions associated with the activation of the extrinsic coagulation pathway. diseases and conditions associated with the activation of the extrinsic coagulation pathway are selected from the group comprising or consisting of thrombosis (including venous and arterial thrombosis), inflammatory diseases with thrombotic tendency, thromboinflammation (including devices-induced thromboinflammation), diseases and conditions associated to cardiac surgical interventions, cardiovascular diseases, cancer and metastasis.

Diseases and conditions associated with the activation of the extrinsic coagulation pathway include the inflammatory diseases with thrombotic tendency. As used herein, the term "inflammatory diseases with thrombotic tendency" may be replaced by "thrombotic complications in inflammatory diseases".

In one embodiment, the invention relates to the use of a protein, a pharmaceutical composition, a medicament or a coated medical device of the invention for treating and/or preventing thrombosis associated with inflammatory disease or thromboinflammation in a subject in need thereof.

In one embodiment, the invention relates to the use of a protein, a pharmaceutical composition, a medicament or a coated medical device of the invention for treating and/or preventing an inflammatory disease with thrombotic tendency or a thromboinflammation selected from the group comprising or consisting of cancer-associated thrombosis, metastasis-associated thrombosis, stroke-associated thrombosis, Behçet's disease (BD), antineutrophil cytoplasmic antibody-associated (ANCA) vasculitides, Takayasu arteritis, rheumatoid arthritis, systemic lupus erythematosus, antiphospholipid syndrome, familial Mediterranean fever, thromboangiitis obliterans (TAO), sepsis, inflammatory bowel diseases, atherosclerosis and plaque rupture, coronary artery disease, acute myocardial infraction, thrombus formation following cerebral injuries, blood-contacting medical devices-induced thromboinflammation, thrombosis induced by catheterism and/or stent positioning procedures at the site of local vascular stenosis, thrombosis induced by extracorporeal circulation, heparin-induced thrombocytopenia (HIT), immunothrombosis, thrombosis associated with preeclampsia, thrombotic complication in cell and cell cluster transplantation and in whole organ transplantation or grafts, venous thromboembolism, aneurysms, and skeletal muscle ischemia-reperfusion syndrome.

In one embodiment, said thromboinflammation is selected from the group comprising or consisting of thrombosis induced by catheterism and/or stent positioning procedures at the site of local vascular stenosis, atherosclerosis, plaque rupture, coronary artery disease, devices-induced thromboinflammation. In one embodiment, devices-induced thromboinflammation includes blood-contacting medical devices-induced thromboinflammation. In another embodiment, said thromboinflammation is selected from the group comprising or consisting of cancer-associated thrombosis, metastasis-associated thrombosis, and stroke-associated thrombosis.

Diseases and conditions associated with the activation of the extrinsic coagulation pathway include blood contacting medical devices-induced thromboinflammation. In one embodiment, blood contacting medical devices-induced thromboinflammations include, but are not limited to, inflammations induced by catheterism (such as, for example, percutaneous transluminal angioplasty) and/or stent positioning procedures at the site of local vascular stenosis, considering the risk of atherosclerotic plaque rupture and subsequent risk of re-thrombosis and catheter thrombosis after such interventions; and extracorporeal circulation.

In one embodiment, the invention relates to the use of a protein or polypeptide, a pharmaceutical composition, a medicament or a coated medical device of the invention for treating and/or preventing devices-induced thromboinflammation in a subject in need thereof.

In one embodiment, the pharmaceutical composition, or medicament of the invention is in a form adapted for topical administration.

Examples of forms adapted for topical administration include, but are not limited to, liquid, paste or solid compositions, and more particularly in form of aqueous solutions, drops, eye drops, ophthalmic solutions, dispersions, sprays, microcapsules, micro- or nanoparticles, polymeric patch, or controlled-release patch.

In one embodiment, the pharmaceutical composition, or medicament of the invention comprises one or more pharmaceutical acceptable carrier for a formulation adapted for topical administration.

In one embodiment, the pharmaceutical composition, or medicament of the invention is in a form adapted for injection, such as, for example, for intraocular, intramuscular, subcutaneous, intradermal, transdermal or intravenous injection or infusion.

Examples of forms adapted for injection include, but are not limited to, solutions, such as, for example, sterile aqueous solutions, dispersions, emulsions, suspensions, solid forms suitable for using to prepare solutions or suspensions upon the addition of a liquid prior to use, such as, for example, powder, liposomal forms and the like.

Sterile injectable forms of the composition, pharmaceutical composition, medicament of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In a particular embodiment, the pharmaceutical composition, or medicament of the invention is in a form adapted for intraocular administration.

In one embodiment, the pharmaceutical composition, or medicament of the invention is administered to the subject in need thereof at least once a day. For example, the pharmaceutical composition, or medicament of the invention may be administered once a day, twice a day, or three times a day. In a preferred embodiment, the pharmaceutical composition, or medicament of the invention is administered to the subject in need thereof once a day.

In another embodiment, the pharmaceutical composition, or medicament of the invention is administered to the subject in need thereof at least once a week. For example, the pharmaceutical composition, or medicament of the invention may be administered once a week, twice a week, three times a week, four times a week or up to seven times a week.

In another embodiment, the pharmaceutical composition, or medicament of the invention is administered to the subject in need thereof once a month, two times a month, every two months, every two or three months, two times a year or once a year.

In one embodiment, the pharmaceutical composition, or medicament of the invention is administered to the subject in need thereof before being exposed to a risk to develop thrombus. In one embodiment, the term "before" means at least a week before the exposure. In another embodiment, the term "before" means five days, four days, three days, two days or one day before the exposure. In another embodiment, the term "before" means 24 hours, 18 hours, 15 hours, 12 hours, 6 hours, 4 hours, 2 hours or 1 hour before the exposure. In another embodiment, the term "before" means less than one hour before the exposure, such as 45 minutes, 30 minutes, 15 minutes, 10 minutes, 5 minutes before the exposure or at the moment of the exposure. As an illustration, the composition, pharmaceutical composition, or medicament of the invention may be administered to the subject 24 hours, 12 hours, 6 hours or 1 hour before a prolonged travel, or at the beginning of the prolonged travel.

In another embodiment, the pharmaceutical composition, or medicament of the invention is administered to the subject in need thereof after being exposed to a risk to develop thrombus. In one embodiment, the term "after" means 5 minutes, 10 minutes, 15 minutes, 30 minutes, or 45 minutes after the exposure. In another embodiment, the term "after" means 1 hour, 2, 4, 6, 12, 15, 18 or 14 hours after the exposure. In another embodiment, the term "after" means 1 day, 2, 3, 4 or five days after the exposure. In another embodiment, the term "after" means a week or more after the exposure. As an illustration, the pharmaceutical composition, or medicament of the invention may be administered to the subject immediately after a prolonged travel, 1 hour, 2 hours, 6 hours, or 12 hours after the prolonged travel.

In one embodiment, the coated medical device coated with the protein of the invention is formulated for administration to the subject. The coated medical device coated with the protein of the present invention may be administered parenterally or topically, or may implanted.

The present invention also relates to a kit comprising a protein or polypeptide, pharmaceutical composition, or medicament or coated medical device according to the invention.

In one embodiment, the kit of the invention further comprises means to administer the protein or polypeptide, pharmaceutical composition, medicament or coated medical device to a subject in need thereof.

In one embodiment, the kit of the invention further comprises instructions for the administration of the protein or polypeptide, pharmaceutical composition, medicament or coated medical device to said subject.

### BRIEF DESCRIPTION OF THE DRAWINGS

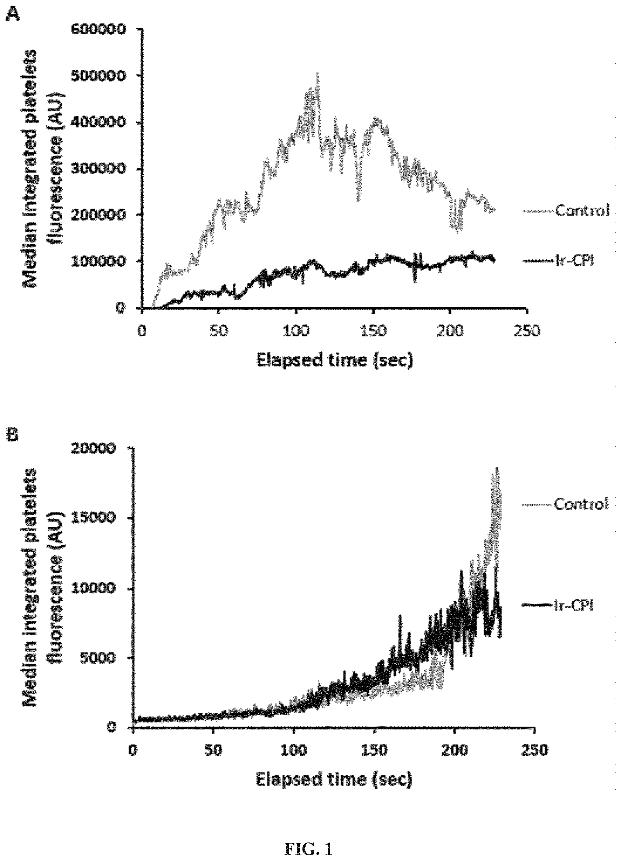

**Figure 1** is a combination of graphs showing the *in vivo* detection of platelets **(A)** and fibrin **(B)** after thrombus formation was induced in mice by vessel wall injury. Fibrin and platelets were detected using an Alexa Fluor 488-conjugated anti-fibrin antibody and a DyLight649-labeled anti-GPIb antibody, respectively. The median of integrated fluorescence over time (short time period) is presented for 53 (control) and 54 (Ir-CPI) thrombi generated in 5 mice per group. AU: arbitrary units.

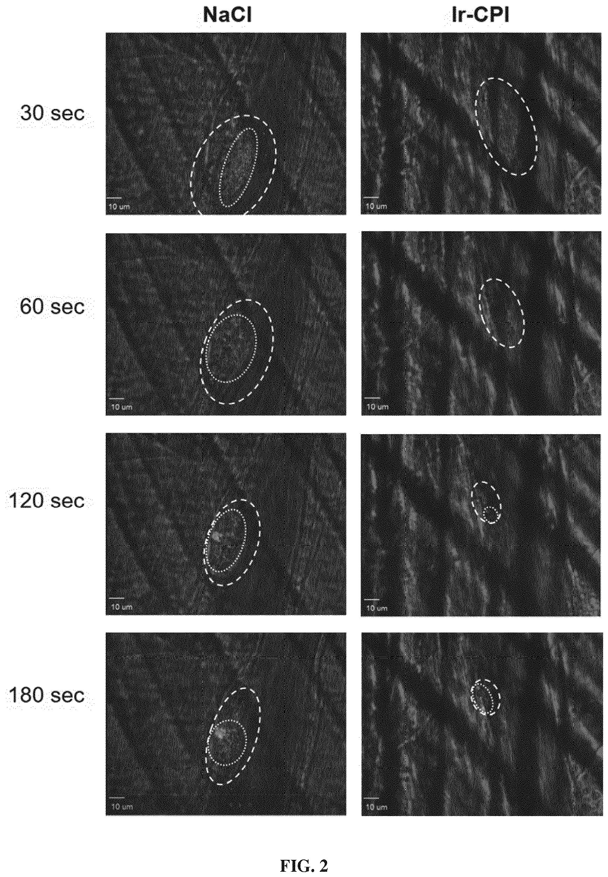

**Figure 2** is a combination of representative images of fluorescence microscopy taken *in vivo* 30, 60, 120 and 180 seconds after laser injury of a vessel wall inducing the formation of thrombus in mice treated with NaCl (control) or Ir-CPI. Fibrin and platelets were detected using an Alexa Fluor 488-conjugated anti-fibrin antibody and a DyLight649-labeled anti-GPIb antibody, respectively. Areas surrounded by a dashed line depict platelet accumulation and areas surrounded by a dotted line depict fibrin accumulation. Bars: 10 µm.

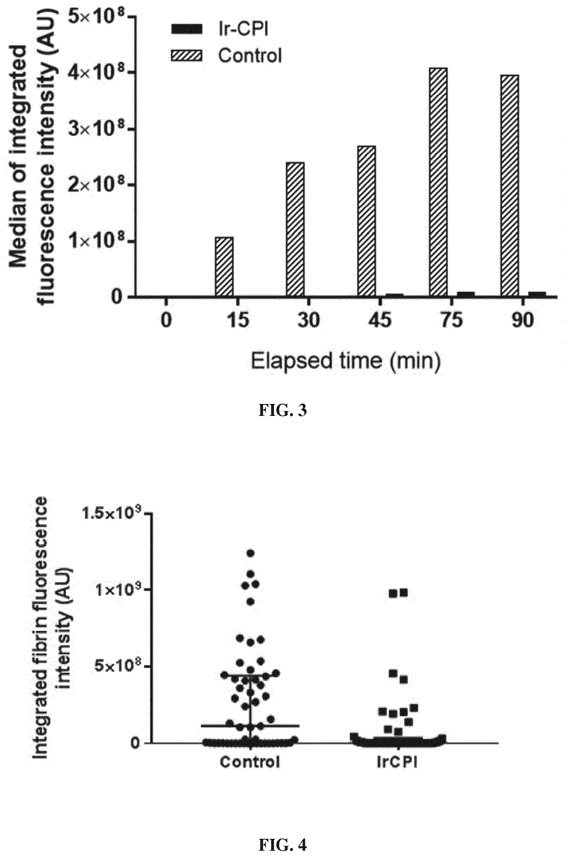

**Figure 3** is a graph showing the *in vivo* detection of fibrin during a 90 minute-elapsed time after thrombus formation was induced in mice by vessel wall injury. Fibrin was detected using an Alexa Fluor 488-conjugated anti-fibrin antibody. The median of integrated fluorescence over time (long time period) is presented for 53 (control) and 50 (Ir-CPI) thrombi generated in 9 mice (n=9 mice per group). AU: arbitrary units.
**Figure 4** is a graph showing the *in vivo* detection of fibrin during a 90 minute-elapsed time after thrombus formation was induced in mice by vessel wall injury. Fibrin was detected using Alexa Fluor 488-conjugated anti-fibrin antibody. Medians with inter-quartile ranges are presented for the control (n=53 thrombi) and Ir-CPI-treated mice (n=50 thrombi) generated in 9 mice (n=9 mice per group). AU: arbitrary units.

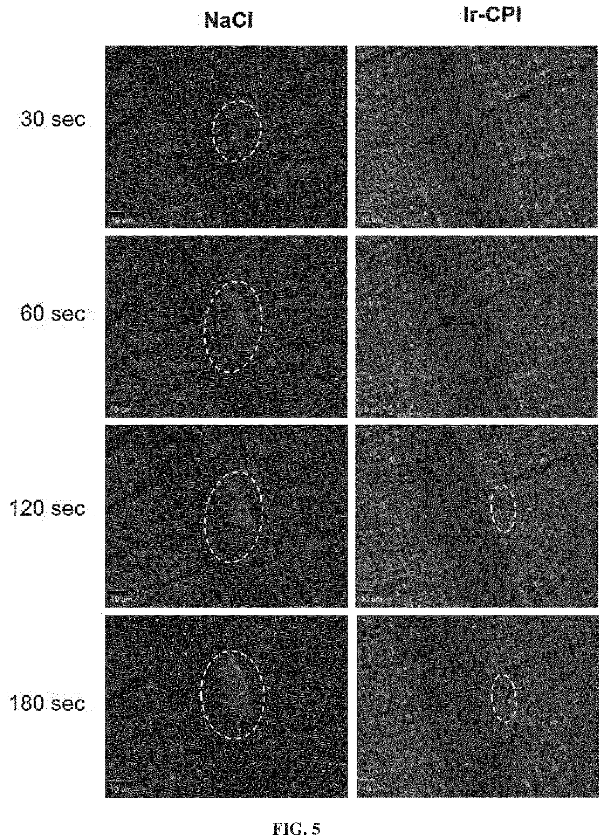

**Figure 5** is a combination of representative images of fluorescence microscopy taken *in vivo* 30, 60, 120 and 180 seconds after laser injury of a vessel wall inducing the formation of thrombus in mice treated with NaCl (control) or Ir-CPI. PMNs were detected using a PE-labelled anti-Ly6G antibody. Areas surrounded by a dashed line depict PMN accumulation. Bars: 10 µm.

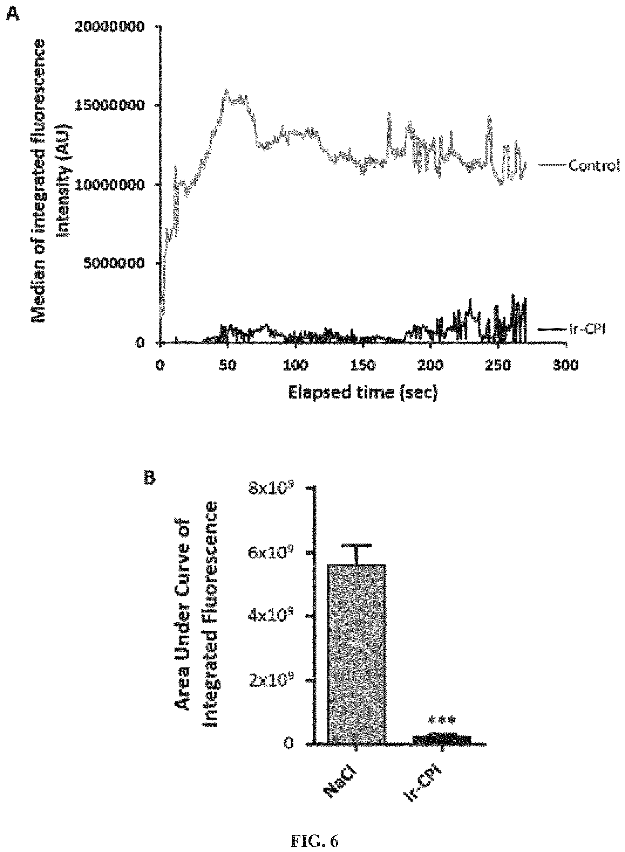

**Figure 6** is a combination of graphs showing the *in vivo* detection of PMNs after thrombus formation was induced in mice by vessel wall injury. PMN accumulation was assessed by using a PE-labeled anti-Ly6G antibody. The median of integrated fluorescence over time **(A)** and the corresponding area under curve **(B)** are presented for 26 (control) or 27 (Ir-CPI) thrombi generated in 8 mice (n=4 mice per group). AU: arbitrary units. A Mann-Whitney test was used for statistical analysis (***p<0.001).

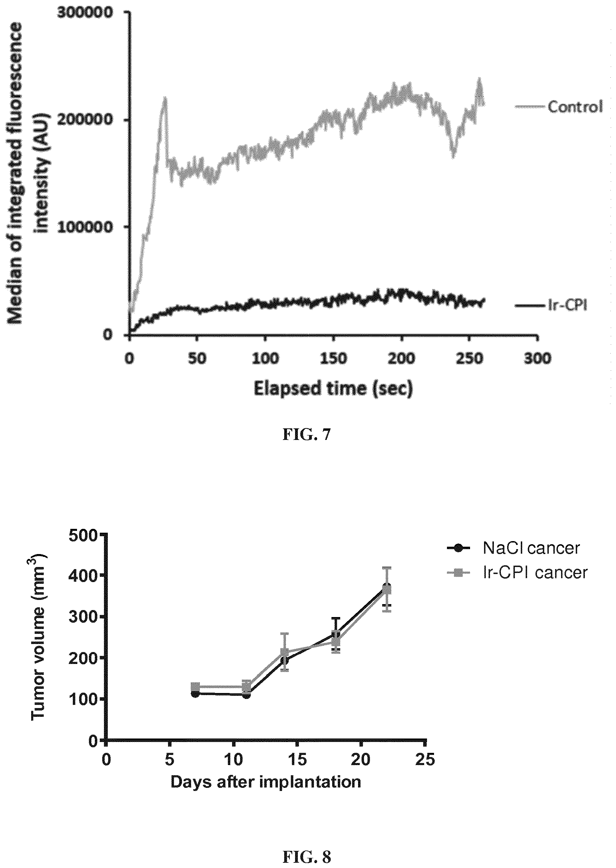

**Figure 7** is a graph showing the *in vivo* detection of neutrophil elastase activity after thrombus formation was induced in mice by vessel wall injury. Neutrophil elastase activity was assessed by using a BODIPY-FL-labeled DQ-elastin conjugate fragment as fluorescently labeled substrate of elastase. The median of integrated fluorescence over time is presented for 31 (control) or 23 (Ir-CPI) thrombi generated in 7 mice (n=3 for control mice and n=4 for Ir-CPI-treated mice). AU: arbitrary units.
**Figure 8** is a graph showing the tumor volume after implantation of murine Panc02 cells in mice. Before the laser injury experiments, tumor volume was evaluated in mice bearing ectopic pancreatic tumors induced by Panc02 cells. In black, mice dedicated to control treatment, *i*.*e*., NaCl, (n=5) and in grey, mice dedicated to Ir-CPI treatment (n=4). Mean tumor volumes ± SEM are represented.

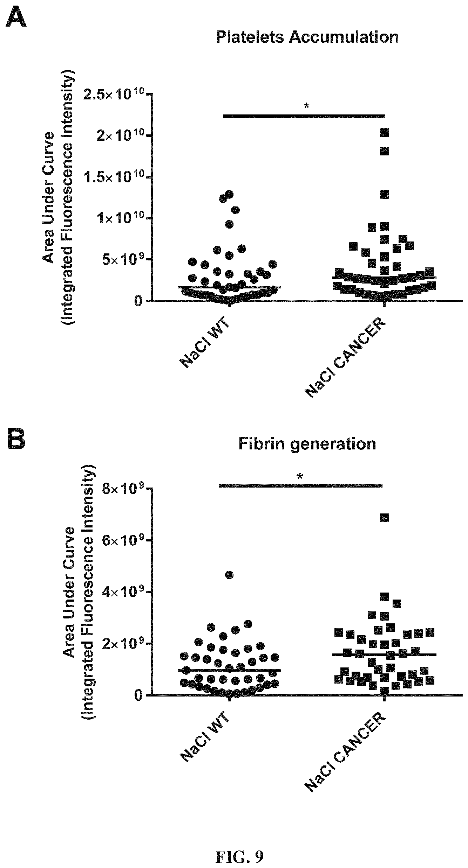

**Figure 9** is a combination of graphs showing the *in vivo* detection of platelets and the generation of fibrin after thrombus formation was induced in mice by vessel wall injury. Fibrin and platelets were detected using an Alexa Fluor 488-conjugated anti-fibrin antibody and a DyLight649-labeled anti-GPIb antibody. The area under curve of platelet **(A)** or fibrin integrated fluorescence intensity **(B)** is presented for 41 (NaCl WT, i.e. cancer-free; n = 3 mice) or 40 (NaCl cancer; n= 6 mice) thrombi generated. A Mann-Whitney test was used for statistical analysis (*p<0.05).

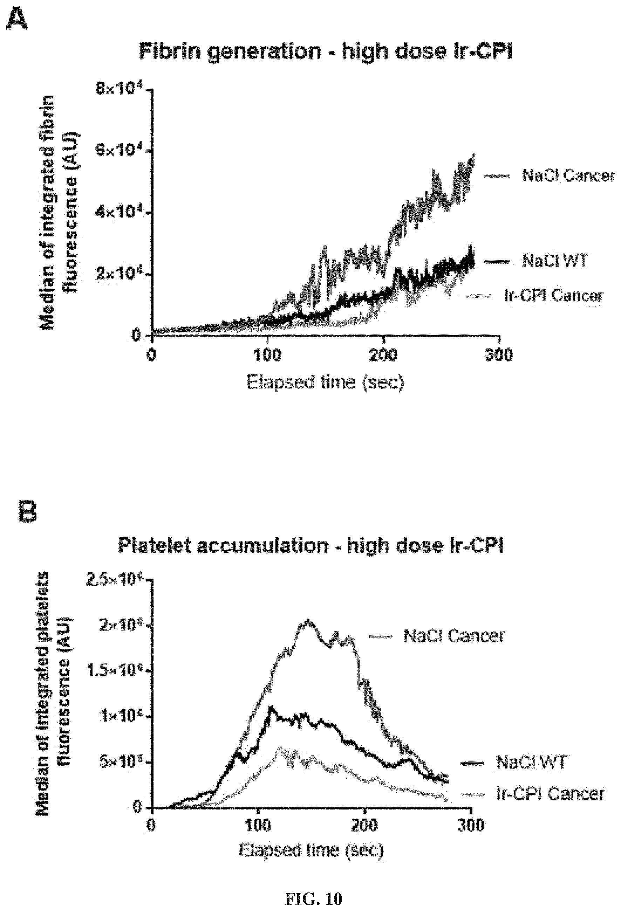

**Figure 10** is a combination of graphs showing the *in vivo* detection of fibrin **(A)** and platelets **(B)** after thrombus formation was induced in tumor-bearing mice by vessel wall injury in mice treated with 20.5 mg/kg (bolus) + 3.7 mg/kg/h (infusion) (*i.e.* high dose). Fibrin and platelets were detected using an Alexa Fluor 488-conjugated anti-fibrin antibody and a DyLight649-labeled anti-GPIb antibody, respectively. The median of integrated fluorescence over time is presented for 44 (NaCl cancer-free, corresponding to the administration of NaCl to cancer-free mice), 34 (NaCl cancer, corresponding to the administration of NaCl to tumor-bearing mice) and 39 (Ir-CPI cancer, corresponding to the administration of Ir-CPI to tumor-bearing mice) thrombi generated in 14 mice (n=5 for NaCl cancer-free mice, n=5 for NaCl cancer mice and n=4 for Ir-CPI cancer mice). AU: arbitrary units.

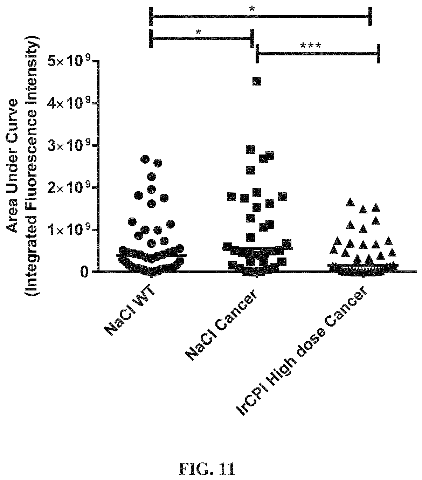

**Figure 11** is a graph showing the integrated fluorescence intensity of platelets (area under curve) presented for 44 (NaCl cancer-free mice), 34 (NaCl cancer mice) or 39 (Ir-CPI cancer mice) thrombi generated in 14 mice treated with high dose Ir-CPI (n=5 for NaCl cancer-free mice, n=5 for NaCl cancer mice; and n=4 for Ir-CPI cancer mice). Platelets were detected using a DyLight649-labeled anti-GPIb antibody. A Mann-Whitney test was used for statistical analysis (*p<0.05; ***p<0.001).

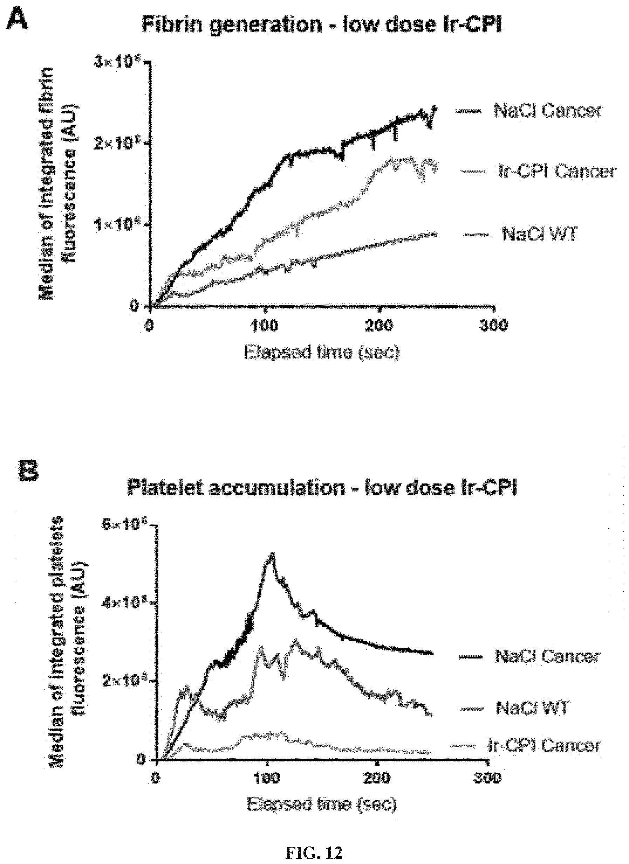

**Figure 12** is a combination of graphs showing the *in vivo* detection of fibrin **(A)** and platelets **(B)** after thrombus formation was induced in tumor-bearing mice by vessel wall injury in mice treated with 10.3 mg/kg (bolus) + 1.9 mg/kg/h (infusion) (*i.e.* low dose). Fibrin and platelets were detected using an Alexa Fluor 488-conjugated anti-fibrin antibody and a DyLight649-labeled anti-GPIb antibody, respectively. The median of integrated fluorescence over time is presented for 41 (NaCl cancer-free, corresponding to the administration of NaCl to cancer-free mice), 40 (NaCl cancer, corresponding to the administration of NaCl to tumor-bearing mice) and 40 (Ir-CPI cancer, corresponding to the administration of Ir-CPI to tumor-bearing mice) thrombi generated in 14 mice (n=3 for NaCl cancer-free mice, n=6 for NaCl cancer mice and n=5 for Ir-CPI cancer mice). AU: arbitrary units.

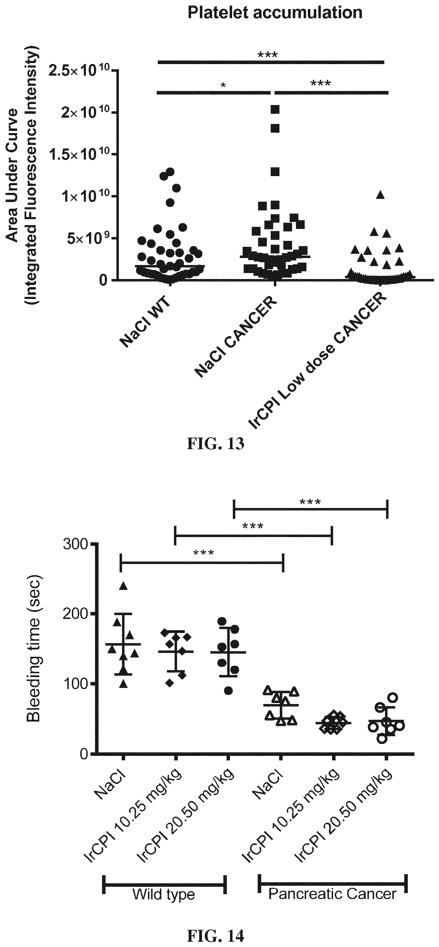

**Figure 13** is a graph showing the integrated fluorescence intensity of platelets (area under curve) presented for 41 (NaCl cancer-free mice), 40 (NaCl cancer mice) or 40 (Ir-CPI cancer mice) thrombi generated in 14 mice (n=3 for NaCl cancer-free mice, n=6 for NaCl cancer mice; and n=5 for Ir-CPI cancer mice). Ir-CPI cancer mice were treated with 10.3 mg/kg (bolus) + 1.9 mg/kg/h (infusion) of Ir-CPI (*i.e.* low dose). Platelets were detected using a DyLight649-labeled anti-GPIb antibody. A Mann-Whitney test was used for statistical analysis (*p<0.05; ***p<0.001).
**Figure 14** is a graph showing bleeding times (in seconds) in NaCl wild-type (*i.e.* cancer-free) mice, in Ir-CPI-treated wild-type (*i.e.* cancer-free) mice (low and high dose) and in NaCl cancer-bearing mice or in Ir-CPI-treated cancer-bearing mice (low and high dose). A one-way ANOVA was used for statistical analysis (***p<0.001). All groups tested included 7 mice excepted for the NaCl wild-type mice group which included 8 mice.

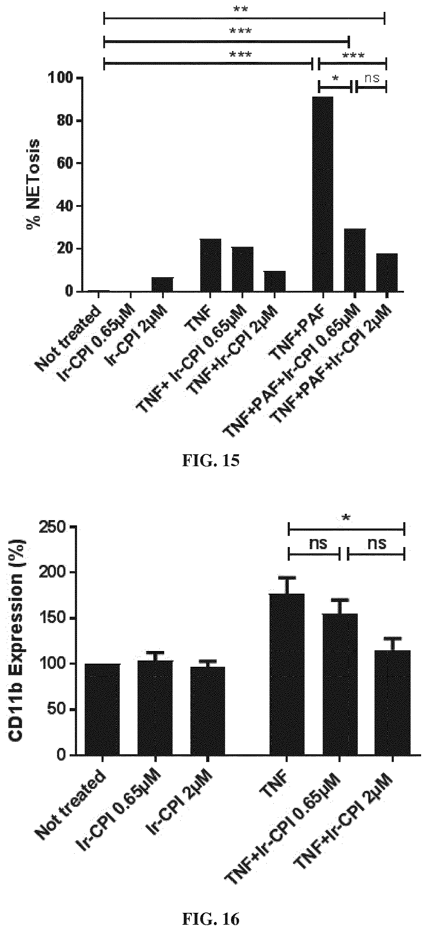

**Figure 15** is a graph comparing NETosis under different *in vitro* experimental conditions (untreated control, priming with tumor necrosis factor α (TNFα) with or without activation with platelet-activating factor (PAF), and/or incubation with Ir-CPI as indicated). The percentage of NETosis (median) was calculated after analyzing immunofluorescent staining and NET length (n=5 independent experiments; 5 random fields per condition were analyzed). A Wilcoxon test was used for statistical analysis (ns: not statistically significant; *p<0.05; **p<0.01; ***p<0.001).
**Figure 16** is a graph comparing the expression of CD11b at the surface of neutrophils under different experimental conditions (untreated control, priming with TNFα and/or incubation with Ir-CPI as indicated). Data are presented as mean ± SEM (n=3 independent experiments). A one-way ANOVA followed by a Tukey's multiple comparison test were used for statistical analysis (ns: not statistically significant; *p<0.05).

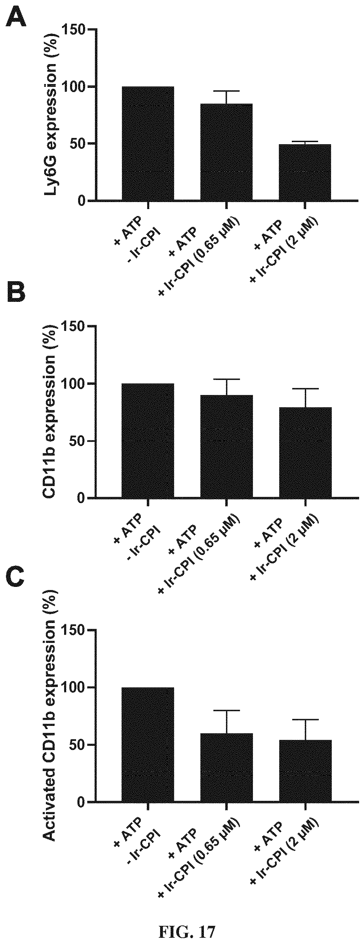

**Figure 17** is a graph comparing the expression of Ly6G **(A),** CD11b **(B),** and activated CD11b **(C)** at the surface of neutrophils under different experimental conditions (control activated by ATP, neutrophils incubated with Ir-CPI [0.65 or 2 µM] and activated with ATP). Data are presented as mean ± SEM (n=2 independent experiments).

### EXAMPLES

The present invention is further illustrated by the following examples.

### Example 1: Model involving the activation of the extrinsic coagulation pathway

### Materials and Methods

### Material

### Test substance

Ir-CPI (MW=7660 Da) was obtained from Bachem (powder, batch 1060411, purity: 98.4 %) and stored at -80°C until use.

Ir-CPI was freshly prepared on the testing day by dissolution in NaCl 0.9 % to reach the concentration of 50 mg/mL (corresponding to the maximal concentration to reach to avoid solubility issues).

NaCl 0.9 % was used as a control.

### Animals

Male mice (C57BL/6JRj) purchased from Janvier LABS were used for this study (5 to 8 weeks old).

### Methods

### Administration

Ir-CPI was administered intravenously as a bolus (20.5 mg/kg) immediately followed by a continuous infusion at a rate of 3.7 mg/kg/h (see **Table 1** below). Due to fast distribution and elimination of Ir-CPI in the mice (t1/2α = 14 min and t1/2β = 236 min), the combination of the bolus and the infusion was chosen in order to maintain a constant Ir-CPI concentration into the blood.

**Table 1: Experiment set-up**

| **Model** | **n** | **Test item** | **Dose bolus (mg/kg)** | **Dose infusion (mg/kg/h)** | **Time of monitoring** |
|---|---|---|---|---|---|
| Laser injury (platelet + fibrin detection) | 5 | Control (NaCl 0.9 %) | - | - | 3.8 min (228 sec) |
| | 5 | Ir-CPI | 20.5 | 3.7 | 3.8 min (228 sec) |
| Laser injury (kinetics of fibrin generation) | 9 | Control (NaCl 0.9 %) | - | - | 90 min |
| | 9 | Ir-CPI | 20.5 | 3.7 | 90 min |
| Laser injury (platelets + polymorphonuclear neutrophils) | 4 | Control (NaCl 0.9 %) | - | - | 4.5 min (270 sec) |
| | 4 | Ir-CPI | 20.5 | 3.7 | 4.5 min (270 sec) |
| Laser injury (assessment of neutrophil elastase activity) | 3 | Control (NaCl 0.9 %) | - | - | 4.3 min (260 sec) |
| | 4 | Ir-CPI | 20.5 | 3.7 | 4.3 min (260 sec) |

| | | | | | |
|---|---|---|---|---|---|
| n= number of mice used. | | | | | |

### Intravital microscopy of the cremaster muscle microcirculation

Intravital video-microscopy of the cremaster muscle microcirculation was done as previously described (Falati *et al.,* 2002).

Mice were anesthetized with intraperitoneal injection of ketamine (125 mg/kg), xylazine (12.5 mg/kg) and atropine (0.25 mg/kg) and were maintained at 37°C on a thermo-controlled rodent blanket.

A tracheal tube was inserted into the trachea to facilitate breathing. A canula was inserted into a jugular vein for administration of pentobarbital. Pentobarbital (12 mg/kg) was administered in the jugular vein every 20-30 minutes to maintain the mice under anesthesia throughout the handling. The same jugular vein canula was used for the administration of the antibodies and the enzymatic substrate coupled to a fluorochrome that is necessary to follow the development of the thrombus (detection of fibrin and/or platelets and/or neutrophils and/or neutrophil elastase activity). A canula was also inserted in the second contralateral jugular vein for administration of the vehicle or the test item (Ir-CPI or NaCl).

After the scrotum was incised, the testicle and surrounding cremaster muscle were exteriorized onto an intravital microscopy tray. The cremaster muscle was continuously perfused with thermo-controlled (37°C) and aerated (95 % N₂, 5 % CO₂) bicarbonate-buffered saline solution throughout the experiment.

Microvessel data were obtained using an Olympus AX61WI microscope with a 60×0.9-numerical aperture water immersion objective. The digital wide-field fluorescence microscopy system has previously been described (Falati et al., 2002). Digital images were captured with a Cooke Sensicam CCD camera in 640 × 480 format.

### Laser-induced injury

Vessel wall injury was induced with a Micropoint Laser System (Photonics Instruments) focused through the microscope objective, parfocal with the focal plane and aimed at the vessel wall, as previously described (Dubois et al., 2006). Typically, 1 or 2 pulses were required to induce vessel wall injury.

Multiple thrombi were studied in a single mouse, with new thrombi formed upstream of earlier thrombi to avoid any contribution from thrombi generated earlier in the animal under study. Laser-beam injuries were done at various time after the start of test-item administration (+ 5 min up to about 150 min after bolus administration of Ir-CPI or NaCl (control)).

Image analysis was performed using Slidebook 4.1 (Intelligent Imaging Innovations). Fluorescence data were captured digitally at up to 50 frames per second and analyzed as previously described (Falati et al., 2002). The kinetics of thrombus formation were analyzed by determining median fluorescence values over time in a minimum number of 20 thrombi.

After the procedure, mice were euthanized with an overdose of pentobarbital (120 mg/kg).

### Detection of fibrin, platelets, PMNs and neutrophil elastase activity in vivo

*In vivo* detection of fibrin was performed using an Alexa Fluor 488-conjugated anti-fibrin antibody from Darbousset et al., 2014, administered intravenously at 0.5 µg/g of mouse.

*In vivo* detection of platelets was performed using a DyLight649-labeled anti-GPIb antibody from Emfret, administered intravenously at 0.5 µg/g of mouse.

Detection of PMNs *in vivo* was performed using PE-labelled anti-Ly6G antibody.

*In vivo* detection of neutrophil elastase activity was performed using BODIPY-FL-labeled DQ-elastin conjugate fragment from Life Technologies, administered intravenously at 0.3 µg/g of mouse.

### Blood sampling and plasma preparation

At the end of the experiment (about 150 min after bolus administration), blood was collected from the inferior vena cava and was placed in 0.109 M 3.2 % sodium citrate tubes (citrate/blood, v/v 1/9). Plasma (around 250 µL) was centrifuged at 18-22°C fat 2500 g during 15 min. Plasma was centrifuged again (18-22°C at 2500 g during 15 min) and was stored into a polypropylene tube at -80°C. Plasma samples were later sent frozen on dry ice for dosage of the circulating concentration of Ir-CPI by ELISA.

### Quantification of Ir-CPI in plasma samples

The quantification of circulating Ir-CPI in the plasma samples was assessed by ELISA at Eurofins, ADME Bioanalyses (Vergèze, France). A sandwich immuno-assay was carried out using two anti-Ir-CPI antibodies. The capture monoclonal antibody (Ir-CPI3) was coated to microtitration plates (Nunc, ThermoFischer Scientific, USA) at 1 µg/mL overnight at 4°C in PBS (Hyclone, USA). Wells were blocked with PBS BSA 2 % (Sigma-Aldrich, USA) at room temperature (RT) for 1h and were then washed. Samples were added into the wells and incubated for 2h at RT. The biotinylated detection antibody (L39-biotin), previously diluted in PBS BSA 1 % (Sigma-Aldrich, USA), was added to the wells and complemented with streptavidin-HRP (REF) diluted in PBS BSA 1 %. TMB SureBlue (KPL, REF) and H₂SO₄ 1N (VWR, USA) were used for spectrophotometric detection (450 nm).

### Data processing and statistical analysis

Data were expressed as median and inter-quartile ranges with associated sample size (n). Statistical comparison of data from Ir-CPI-treated groups versus those from the control group was conducted using a Mann-Whitney test (***p<0.001).

### Results

The goal of the present study was to assess the antithrombotic efficacy of Ir-CPI in an experimental model of thrombosis in mice, wherein thrombosis was induced by a local injury of a cremaster muscle arteriole with a laser beam. This experimental model of thrombosis is thought to involve the activation of the extrinsic coagulation pathway leading to Tissue Factor-mediated thrombin generation (Dubois et al., 2007; Darbousset et al., 2012). Indeed, in such a model, the kinetics of thrombin formation and fibrin generation were reported to be drastically reduced in low Tissue Factor (TF) mice whereas the absence of factor XII (FXII^{-/-} mice) had no effect (Darbousset et al., 2012). The laser-induced vessel wall injury model thus allows to assess the inhibitory effect of Ir-CPI on thrombosis involving the extrinsic coagulation pathway.

### Monitoring of platelet and fibrin accumulation after laser injury in control and Ir-CPI-treated mice (short-time period measurement)

Platelet accumulation and fibrin generation on the injured vessel walls were monitored using the following fluorescently labeled antibodies: DyLight649-labeled anti-GPIb antibody and Alexa Fluor 488-conjugated anti-fibrin antibody, respectively.

In control mice, platelets accumulated rapidly at the site of injury to reach a peak at about T114 sec (median of integrated platelet fluorescence: 508 541 AU) and, then, decreased until the end of the measurement period at T228 sec (median of integrated platelet fluorescence: 209 893 AU). Fibrin generation at the site of injury increased continuously until the end of the measurement period and the median of integrated fibrin fluorescence was 16 686 AU at T228 sec (**Figure 1** and **Figure 2**).

In Ir-CPI-treated mice, the kinetics of platelet accumulation were different to those of control mice. Platelet accumulation was drastically decreased when compared to control mice. The maximal median of integrated platelet fluorescence measured was 121 783 AU at T214 sec. At T228 sec (*i.e.,* the end of the measurement period), the median of integrated platelet fluorescence was 101 887 AU. Moreover, fibrin generation appeared to be less important in Ir-CPI-treated mice than in control mice during the first 3.8 min after laser injury. At T228 sec, the maximal median of integrated fibrin fluorescence was 7 539.7 AU **(****Figure 1** and **Figure 2****).**

Mean plasma concentration measured in Ir-CPI-treated mice after about 150 min of procedure was 6.48 ± 1.02 µg/mL (Mean ± SEM; n = 5).

### Kinetics of fibrin generation after laser injury in control and Ir-CPI-treated mice (long-time period measurement)

The kinetics of fibrin generation were measured during 90 min after vessel wall laser injury in control and Ir-CPI-treated mice. At every time point measured (0, 15, 30, 45, 75 and 90 min), the median of integrated fibrin fluorescence intensity was lower in Ir-CPI-treated mice than in control mice **(****Figure 3** and **Figure 4****).**

### Effect of Ir-CPI on neutrophil accumulation

It was demonstrated that polymorphonuclear neutrophils (PMNs) are the first cells to accumulate to the vessel wall following a laser-induced injury. Interactions of PMNs with activated endothelial cells is required for the initiation of thrombus formation (Darbousset et al., 2012).

To determine whether Ir-CPI has an impact on the accumulation of PMNs at the site of injury, the accumulation of PMNs on the injured vessel wall was monitored using a PE-fluorescently labeled antibody directed against Ly6G **(****Figure 5****).**

In control mice, PMNs accumulated rapidly at the site of injury to reach a peak at T 42 sec (median of integrated platelet fluorescence: 1.60 × 10⁷ AU). Median of integrated PMN fluorescence was 1.10 × 10⁷ AU at T270 sec **(****Figure 6****).**

In Ir-CPI-treated mice, the kinetics of neutrophil accumulation were different to those of control mice. Indeed, during the first 4.5 min after laser injury, PMN accumulation was drastically decreased when compared to control mice. The maximal median of integrated platelet fluorescence measured was 3.02 × 10⁶ AU at T 261 sec. At T 270 sec (*i.e.,* the end of the measurement period), the median of integrated PMN fluorescence was 2.78 × 10⁶ AU **(****Figure 6****).** Similar results were obtained when using a fluorescently labeled substrate of elastase as a marker of the activity of the PMNs. The signal corresponding to neutrophil elastase activity was drastically reduced in Ir-CPI-treated mice when compared to control mice **(****Figure 7****).**

### Conclusion

Taken together, these results demonstrate that Ir-CPI is unexpectedly active in an experimental model that is known to involve the activation of the extrinsic coagulation pathway. These results indicate that Ir-CPI exerts antithrombotic effects that are not necessarily associated to its known targets (*i.e.* Factors XIa and XIIa). Surprisingly, Ir-CPI inhibits at the site of lesion not only the formation of fibrin but also the accumulation of platelets, and even the earlier appearance of PMNs known to play an important role in the activation of the extrinsic coagulation system as they are the main source of TF required for thrombus formation at the site of laser injury (Darbousset, Thomas et al. 2012).

### Example 2: In vitro platelet aggregation assay

### Materials and Methods

Blood from healthy human donors, who had not taken anti-platelet agents within the 15 days prior to collection, was collected in citrated tubes and centrifuged for 13 min at 200g. The platelet-rich plasma was collected and centrifuged for 13 min at 900g in Tyrode buffer (138 mM NaCl, 2.9 mM KCl, 12 mM NaHCO₃, 5.5 mM glucose, 1.8 mM CaCl₂ and 0.4 mM MgCl₂, pH 7.4) containing 0.2 % BSA, apyrase 0.02 U/mL and PGI₂ 500 nM. Washed platelets were resuspended in the Tyrode/BSA 0.2 %/apyrase/PGI₂ solution and centrifuged at 900g for 13 min. Platelets were resuspended in a Tyrode/BSA 0.2 % solution at a concentration of 3.10⁸ platelets/mL. Platelets were kept at 37°C for 30 min in a solution containing 4 µM of calcium and 6 µM of magnesium. Next, platelets were incubated at 37°C under agitation for 5 to 10 minutes in an aggregometer APACT 4004 with ADP (adenosine diphosphate), TRAP (Thrombin Receptor-Activating Peptide) and/or Ir-CPI.

### Results

The effect of Ir-CPI on platelet aggregation was assessed *in vitro* by using isolated human washed platelets (n=1).

Washed platelets were first either incubated with ADP (adenosine diphosphate), an activator of platelets that does not induce platelet aggregation, or with TRAP (Thrombin Receptor Activating Peptide), an activator of platelet aggregation. As expected and as shown in **Table 2** below, ADP did not induce platelet aggregation (max aggregation: 6.9 %) while TRAP activated platelet aggregation (max aggregation: 58.6 to 59.5 %).

**Table 2: Analysis of the effect of Ir-CPI on human washed platelet aggregation**

| **Condition** | **Comments** | **Aggregation** | | |
|---|---|---|---|---|
| | | **Max (%)** | **Slope (%/min)** | **Latency (sec)** |
| TRAP 20 µM | - | 59.5 | 33.1 | 41.0 |
| TRAP 20 µM + Ir-CPI 500 µg/100 µL | TRAP and Ir-CPI injected together | 71.8 | 35.1 | 98.6 |
| Ir-CPI 500 µg/100 µL + TRAP 20 µM | Ir-CPI injected alone and then TRAP injected at about 230 sec | 73.5 | 31.1 | 231.2 |
| TRAP 20 µM | - | 58.6 | 32.4 | 75.4 |
| Ir-CPI 500 µg/100 µL | - | 4.1 | 16.2 | 70.0 |
| ADP 20 µM | - | 6.9 | 19.4 | 51.8 |

Addition of Ir-CPI to washed platelets did not activate platelet aggregation (max aggregation: 4.1 %). The addition of both Ir-CPI and TRAP to washed platelets did not significantly modify platelet aggregation (max aggregation: 71.7 %) when compared to the positive control condition (TRAP alone: max aggregation: 58.6 - 59.5 %). The effect was similar but presented a longer latency period (231.2 sec) when TRAP was added to Ir-CPI (max aggregation: 73.5 %) **(Table 2).**

The results of Example 1 showed that Ir-CPI inhibits platelet accumulation *in vivo.* However, Ir-CPI does not inhibit platelet aggregation in an *in vitro* platelet aggregation assay. Taken together, these results suggest that Ir-CPI may act on another target than platelets, involved in thrombus formation.

In the laser-induced vessel wall injury model, polymorphonuclear neutrophils (PMNs) are known to adhere to injured vessels in the seconds after the lesion, preceding platelets, by binding to the activated endothelium. PMNs, by binding to the vessel wall, are the main source of blood-borne TF in the early phase of thrombus formation (Darbousset et al., 2012). The interaction of PMNs with endothelial cells is a critical step preceding platelet accumulation for thrombosis initiation.

The results of Example 1 show that Ir-CPI decreased PMN recruitment to the injured vessel wall. A reduction of neutrophil elastase activity was also detected under treatment with Ir-CPI. However, this reduction of elastase activity might result from the reduction in the number of neutrophils that have been recruited at the site of the lesion.

### Example 3: Model of cancer-associated thrombosis

### Materials and Methods

### Material

### Test substance

Ir-CPI (MW=7660 Da) was obtained from Bachem (powder, batch 1060411, purity: 98.4 %) and stored at -80°C until use.

Ir-CPI was freshly prepared on the testing day by dissolution in NaCl 0.9 % to reach the concentration of 50 mg/mL (corresponding to the maximal concentration to reach to avoid solubility issues).

### NaCl 0.9 % was used as a control.

### Animals

Male mice (C57BL/6JRj) purchased from Janvier LABS were used for this study (5 weeks old).

### Methods

### Administration

Ir-CPI was administered as indicated hereinabove (see Example 1) and as summarized in **Table 3** below.

**Table 3: Laser injury experiment set-up**

| **Model** | **n** | **Test item** | **Dose bolus (mg/kg)** | **Dose infusion (mg/kg/h)** | **Time of monitoring** |
|---|---|---|---|---|---|
| Laser injury + cancer model **(High dose of Ir-CPI)** | 5 | Control cancer (NaCl 0.9 %) | - | - | 4.6 min (278 sec) |
| | 4 | Ir-CPI cancer | 20.5 | 3.7 | 4.6 min (278 sec) |
| | 5 | Control WT (NaCl 0.9 %) | - | - | 4.6 min (278 sec) |
| Laser injury + cancer model **(Low dose of Ir-CPI)** | 6 | Control cancer (NaCl 0.9 %) | - | - | 4.2 min (250 sec) |
| | 5 | Ir-CPI cancer | 10.3 | 1.9 | 4.2 min (250 sec) |
| | 3 | Control WT (NaCl 0.9 %) | - | - | 4.2 min (250 sec) |

| | | | | | |
|---|---|---|---|---|---|
| n = number of mice used; WT (Wild-Type) corresponds to cancer-free mice. | | | | | |

**Table 4: Tail bleeding time experiment set-up**

| **Model** | **n** | **Test item** | **Dose bolus (mg/kg)** | **Dose infusion (mg/kg/h)** |
|---|---|---|---|---|
| Bleeding time assessment | 8 | Control WT | - | - |
| | 7 | Ir-CPI Low dose WT | 10.3 | 1.9 |
| | 7 | Ir-CPI High dose WT | 20.5 | 3.7 |
| | 7 | Control cancer | - | - |
| | 7 | Ir-CPI Low dose cancer | 10.3 | 1.9 |
| | 7 | Ir-CPI High dose cancer | 20.5 | 3.7 |

| | | | | |
|---|---|---|---|---|
| n = number of mice used; WT (Wild-Type) corresponds to cancer-free mice. | | | | |

### Induction of ectopic tumor

Murine Panc02 cells (mouse pancreatic ductal adenocarcinoma cell line) were cultured to a 80 % confluence in RPMI-1640 medium (Life Technologies) supplemented with 10 % FCS (PAA), 1000 U/mL penicillin (Life Technologies), 100 µg/mL streptomycin (Life Technologies) and 0.1 % fungizone (Life Technologies) at 37°C in a humidified atmosphere with 5 % CO₂. Once in the exponential growth phase, cells were washed three times with PBS and briefly exposed to non-enzymatic cell dissociation buffer (Life Technologies) to dislodge the cells. The cells were carefully washed three times, resuspended in PBS, and diluted to the desired concentration.

Five-week-old C57BL/6 mice were injected subcutaneously in the right flank with a tumor cell suspension (10⁶ cells in 100 µL of PBS). When the tumor became palpable (0.2 cm), measurements in two dimensions were performed with a caliper, and the volume of each tumor was calculated according to the formula for the volume of an ellipsoid: π/6 × a(b)², where a is the largest diameter and b the smallest diameter of the tumor.

### Intravital microscopy of the cremaster muscle microcirculation

Intravital video-microscopy of the cremaster muscle microcirculation was done as previously described (Falati *et al.,* 2002) and as described hereinabove (see Example 1).

### Laser-induced injury

Vessel wall injury was induced with a Micropoint Laser System (Photonics Instruments) as described hereinabove (see Example 1).

Platelet and fibrin accumulation after injury were monitored and detected as indicated hereinabove (see Example 1).

### Tail bleeding time assessment

Tail bleeding time assessment under NaCl or Ir-CPI treatment (Low or High dose) was performed as previously described (Mezouar et al., 2015) on anesthetized wild-type (*i.e.* cancer-free) or cancer-bearing mice. Briefly, a 1- to 3-mm portion of the distal tail was removed from mice; the tail was immersed in isotonic saline (37°C), and the time to complete cessation of blood flow recorded. The bleeding time was monitored for a maximum of 10 min.

### Data processing and statistical analysis

For the tumor volume assessment, data were expressed as mean ± SEM with associated sample size (n). For the assessment of Ir-CPI effect on platelet accumulation and fibrin generation in tumor-bearing mice, data were expressed as the median of integrated fluorescence over time. A Mann-Whitney test was used for statistical analysis and a One-way ANOVA with Tukey's multiple comparison test was used for the tail bleeding time.

### Results

Using the same model of thrombosis induction, the antithrombotic effect of Ir-CPI was assessed in pancreatic cancer-bearing mice (ectopic model). Indeed, it was demonstrated that pancreatic cancer cells produce Tissue Factor (TF)-bearing microparticles (MP) playing a key role in thrombus formation *in vivo* (Thomas et al., 2009; Mezouar et al., 2015). Two doses (high dose and low dose) of Ir-CPI were tested in this model.

In another experiment, wild-type (cancer-free) and cancer-bearing mice, the time for tail bleeding to stop was assessed under NaCl (control) or Ir-CPI treatment (Low and High dose).

### Assessment of tumor volume before laser injury experiments

In the mouse model of the study, cancer was induced by subcutaneous injection of mouse pancreatic (Panc02) cancer cells. Tumor-bearing mice were randomly divided into two groups (control and Ir-CPI). 22 days after the implantation of the Panc02 cells, mice from both groups received an injection of vehicle (NaCl (control)) or test-item (Ir-CPI) and were submitted to laser injuries of the cremaster muscle microcirculation for thrombus induction. As shown on **Figure 8****,** the mean tumor volume in the two groups was not statistically different at day 22 (control: 372.6 ± 45.3 mm³; n=5 and Ir-CPI: 365.0 ± 52.1 mm³; n=4). The latter mice were used for the experiment with High dose of Ir-CPI treatment. Mean tumor volume was also not statistically different in mice dedicated to the treatment with the Low dose of Ir-CPI (data not shown).

### Monitoring of platelet and fibrin accumulation after laser injury

Tumor-bearing mice were attributed to the Ir-CPI-treated group (Ir-CPI cancer) or to the control group (NaCl cancer). Moreover, cancer-free mice treated with NaCl (NaCl cancer-free) were also used as negative control. All mice were submitted to laser injuries in arterioles of the cremaster muscle to trigger thrombosis. Ir-CPI treated mice received either a low or a high dose of the investigative product.

Platelet accumulation and fibrin generation on the injured vessel walls were monitored using the following fluorescently labeled antibodies: DyLight649-labeled anti-GPIb antibody and Alexa Fluor 488-conjugated anti-fibrin antibody, respectively.

NaCl cancer mice presented a prothrombotic state as assessed by the high accumulation of platelets and high amount of fibrin generation at the sites of injuries when compared to the NaCl cancer-free mice. Indeed, the integrated fluorescence intensity of platelet and fibrin labeling (area under curve) was significantly higher in control cancer mice when compared to control WT mice (*p<0.05). In the control cancer and control WT mice, the median of integrated fibrin fluorescence was 59 172 AU and 23 005 AU at T 278 sec, respectively (**Figure 9** and **Figure 10**).

A significant decrease of platelet accumulation and fibrin generation was observed in both High dose and Low dose Ir-CPI-treated cancer mice when compared to the NaCl cancer mice. As shown in **Figure 11****,** the integrated fluorescence intensity of platelet labeling (area under curve) was significantly lower in High dose Ir-CPI-treated cancer mice than in NaCl cancer-free mice (*p<0.05) and NaCl cancer mice (***p<0.001). Similar results were obtained with Low dose Ir-CPI-treated mice (**Figure 12** and **Figure 13**).

### Assessment of tail bleeding time

Tail bleeding time was significantly reduced (***p<0.001) in mice developing a tumor (e.g. median time for NaCl treated-mice that received Panc02 cancer cells was 75 sec) in comparison with mice that did not (e.g. median time for wild-type (*i.e.* cancer-free) NaCl treated-mice was 147 sec) **(****Figure 14****).** No significant effect of Ir-CPI treatment (Low and High dose) was observed on the tail bleeding time in cancer-free and in cancer-bearing mice, as compared to NaCl-treated mice **(****Figure 14****).**

### Conclusion

The pathogenesis of the prothrombotic state in cancer is associated with the generation of a local and systemic hypercoagulable/thrombotic state that confers a growth advantage to tumor cells. It was demonstrated that pancreatic cancer cells produce tissue factor (TF)-bearing microparticles (MP) playing a key role in thrombus formation *in vivo* (Thomas et al., 2009; Mezouar et al., 2015). PMNs in mice with cancer have increased propensity to form neutrophil extracellular traps (NETs) - web-like structures formed by externalized chromatin and secreted proteases; a process called NETosis (Leal et al., 2017). Moreover, extracellular chromatin released through NET formation was demonstrated to be a cause for cancer-associated thrombosis (Demers et al., 2012; Mauracher et al., 2018).

The results of Example 3 show that Ir-CPI inhibits the prothrombotic state linked to cancer in the mouse model of laser injury. Indeed, in mice bearing an ectopic pancreatic tumor, Ir-CPI decreases significantly platelet accumulation and fibrin formation at the sites of laser injury. Importantly, in cancer-free and cancer-bearing mice, Ir-CPI has no effect on the tail bleeding time, showing that it does not impair hemostasis.

### Example 4: NETosis assay

### Materials and Methods

### Material

Neutrophils were isolated from the bone marrow of femurs of mice (C57BL/6) as previously described (Hu 2012) using FR-1-coupled magnetic beads (anti-Ly6G Microbead Kit, Miltenyi Biotec).

### Methods

### In vitro NETosis assay

Cells were harvested, centrifugated and incubated during 30 min with or without 2 ng/mL of TNFα in Hanks solution (Gibco). Neutrophils were seeded on slides coated with poly-L-lysine and incubated at 37°C for 1 h in Hanks solution. Next, neutrophils were activated with or without 25 µM of platelet-activating factor (PAF) ± Ir-CPI (0.65 or 2 µM) in Hanks solution (3h at 37°C). NET formation was assessed by immunofluorescence (fluorescent microscope from Leica) after fixation of neutrophils with 4 % PAF (paraformaldehyde) and fluorescent labelling of citrullinated histones (H3 antibody) and nuclei (Hoechst). Processing and analysis of images was performed using the FIJI software.

### Data processing and statistical analysis

Percentage of NETosis (median) was presented for 5 independent experiments. A Wilcoxon test was used for statistical analysis (ns: not significant; * p < 0.05; ** p < 0.01; *** p < 0.001).

### Results

Following the results of Example 3, the effect of Ir-CPI on the PMN activation state was assessed *in vitro* by assessing NET formation. Indeed, it was reported that PMNs in mice with cancer have increased propensity to form neutrophil extracellular traps (NETs) demonstrated to be a cause for cancer-associated thrombosis (Demers, Krause et al. 2012, Mauracher, Posch et al. 2018).

### Effect of Ir-CPI on the NETosis in vitro

It was reported that PMNs in mice with cancer have an increased propensity to form neutrophil extracellular traps (NETs), *i*.*e*., web-like structures formed by externalized chromatin and secreted proteases, a process called NETosis (Leal et al., 2017). Moreover, extracellular chromatin released through NET formation was demonstrated to be a cause for cancer-associated thrombosis (Demers et al., 2012; Mauracher et al., 2018).

To assess the effect of Ir-CPI on NETosis, immunofluorescent staining (Hoechst for DNA and H3Cit for citrullinated histones labeling) was performed on PMNs isolated from bone marrow of femurs of mice and submitted to different *in vitro* experimental conditions (untreated control, priming with TNFα with or without activation with PAF, and/or incubation with Ir-CPI) as shown on **Figure 15****.**

As positive control of NET formation, PMNs primed with TNFα and activated with PAF were used. These PMNs presented an increased percentage of NETosis when compared to untreated PMNs (*** p < 0.001), which validated the model **(****Figure 15****).**

As shown on **Figure 15****,** when PMNs were primed with TNFα, activated with PAF and incubated with Ir-CPI (0.65 and 2 µM), a significant reduction of the percentage of NET formation was observed when compared to PMNs incubated with TNFα and PAF (* p < 0.05 for Ir-CPI at 0.65 µM and *** p < 0.001 for Ir-CPI at 2 µM).

These results demonstrate that, *in vitro,* Ir-CPI reduces NETosis when incubated with activated neutrophils.

### Example 5: Effect of Ir-CPI on neutrophil activation

### Materials and Methods

Neutrophils were isolated from the bone marrow of femurs of mice (C57BL/6) as previously described (Hu 2012), using FR-1-coupled magnetic beads (anti-Ly6G Microbead Kit, Miltenyi Biotec). For activation with TFNα, cells were harvested, centrifugated and incubated during 30 min with or without 2 ng/mL of TNFα in Hanks solution (Gibco). Next, neutrophils were incubated with Ir-CPI (0.65 or 2 µM) in Hanks solution (1h at 37°C). For activation with ATP, cells were harvested, centrifugated and incubated with ATP 10 µM and Ir-CPI (0.65 or 2 µM) in Hanks solution (1h at 37°C). After washing of cells, expression of CD1 1b, activated CD1 1b or Ly6G was assessed by flow cytometry (Beckman Coulter Gallios). Antibodies used for neutrophil identification and activation analyses were IRR IgG1-FITC (irrelevant antibody), anti-CD45-APC, anti-Ly6G-PE and anti-CD11b-FITC or Alexa Fluor 647 anti-activated CD11b-. Analysis of results was performed using the Kaluza software (Beckman Coulter).

### Data processing and statistical analysis

Percentage of activation marker expression was presented as mean ± SEM (n=3 independent experiments for TNFα activation and n=2 independent experiments for ATP activation). For experiments with TNFα activation, a one-way ANOVA followed by a Tukey's multiple comparison test were used for statistical analysis (ns: not significant; * p < 0.05).

### Results

Following the results of Example 4, the effect of Ir-CPI on the PMN activation state was assessed *in vitro* by assessing the expression of neutrophil activation markers following incubation with molecules known to activate neutrophils, namely Adenosine Triphosphate (ATP) and Tumor Necrosis Factor α (TNFα).

ATP was shown to contribute to neutrophil activation leading to their adhesion at the site of laser-induced endothelial injury, a necessary step leading to the generation of fibrin, and subsequent platelet-dependent thrombus formation (Darbousset, Delierneux et al. 2014). Moreover, TNFα, another activator of neutrophils (Futosi, Fodor et al. 2013), was tested in this example.

### Effect of Ir-CPI on neutrophil activation following incubation with TNFα

Neutrophils isolated from bone marrow of femurs of mice were submitted to different *in vitro* experimental conditions (untreated control, priming with TNFα and/or incubation with Ir-CPI) to assess the effect of Ir-CPI on their activation status. The activation status was assessed by analyzing the percentage of CD11b expression at the surface of neutrophils **(****Figure 16****).**

Incubation of untreated neutrophils with Ir-CPI (0.65 and 2 µM) had no effect on the expression of CD11b. Neutrophils primed with TNFα were used as positive control of neutrophil activation. As shown on **Figure 16****,** neutrophils primed with TNFα presented an increased expression of CD11b when compared to untreated neutrophils. Strikingly, neutrophils primed with TNFα and incubated with Ir-CPI (2 µM) displayed a significant reduction of the CD11b expressed at their cell surface (* p < 0.05).

These results demonstrate that Ir-CPI alters neutrophil activation induced by TNFα through a decreased CD11b expression *in vitro.*

### Effect of Ir-CPI on neutrophil activation following incubation with ATP

Neutrophils isolated from bone marrow of femurs of mice were submitted to different *in vitro* experimental conditions (untreated control or incubation with Ir-CPI, and with or without ATP) to assess the effect of Ir-CPI on their activation status. The activation status was assessed by analyzing the percentage of CD11b, activated CD11b and Ly6G expressions at the surface of neutrophils **(****Figure 17****).**

Incubation of untreated neutrophils with Ir-CPI (0.65 and 2 µM) had no effect on the expression of CD11b **(****Figure 17B****).** Nevertheless, Ir-CPI significantly decreased the expression of activated CD11b at the surface of neutrophils **(****Figure 17C****).** The expression of Ly6G was also decreased at the surface of neutrophils when incubated with Ir-CPI

### (Figure 17A).

These results demonstrate that, *in vitro,* Ir-CPI alters neutrophil activation induced by ATP through a decreased expression of activated CD11b.

Thus, Ir-CPI inhibits the thrombus formation following activation of the extrinsic coagulation pathway not through the inhibition of tissue factors specific of this pathway, such as Tissue Factor, but through the inhibition of neutrophil recruitment, activation and NETs formation. These events are the initial steps resulting in the activation of the extrinsic coagulation system and subsequent thrombus formation following laser endothelial injury.

### REFERENCES

Darbousset, R., et al. (2014). "P2X1 expressed on polymorphonuclear neutrophils and platelets is required for thrombosis in mice." Blood. 124(16):2575-2585.
Darbousset, R., et al. (2012). "Tissue factor-positive neutrophils bind to injured endothelial wall and initiate thrombus formation." Blood. 120(10):2133-2143.
Decrem, Y., et al. (2009). "Ir-CPI, a coagulation contact phase inhibitor from the tick Ixodes ricinus, inhibits thrombus formation without impairing hemostasis." J Exp Med. 206(11):2381-2395.
Demers, M., et al. (2012). "Cancers predispose neutrophils to release extracellular DNA traps that contribute to cancer-associated thrombosis." Proc Natl Acad Sci U S A. 109(32): 13076-13081.
Dubois, C., et al. (2007). "Thrombin-initiated platelet activation in vivo is vWF independent during thrombus formation in a laser injury model." J Clin Invest; 117(4):953-960.
Dubois, C., et al. (2006). "Glycoprotein VI-dependent and -independent pathways of thrombus formation in vivo." Blood. 107(10):3902-3906.
Falati, S., et al. (2002). "Real-time in vivo imaging of platelets, tissue factor and fibrin during arterial thrombus formation in the mouse." Nat Med. 8(10):1175-1181.
Futosi, K., et al. (2013). "Neutrophil cell surface receptors and their intracellular signal transduction pathways." Int Immunopharmacol. 17(3):638-650.
Hu, Y. (2012). "Isolation of human and mouse neutrophils ex vivo and in vitro." Methods Mol Biol. 844:101-113.
Kambas, K., et al. (2012). "The emerging role of neutrophils in thrombosis-the journey of TF through NETs." Front Immunol. 3:385.
Keularts, I. M., et al. (2001). "The role of factor XI in thrombin generation induced by low concentrations of tissue factor." Thromb Haemost. 85(6):1060-1065.
Leal, A. C., et al. (2017). "Tumor-Derived Exosomes Induce the Formation of Neutrophil Extracellular Traps: Implications For The Establishment of Cancer-Associated Thrombosis." Sci Rep. 7(1):6438.
Massberg, S., et al. (2010). "Reciprocal coupling of coagulation and innate immunity via neutrophil serine proteases." Nat Med. 16(8):887-896.
Mauracher, L. M., et al. (2018). "Citrullinated histone H3, a biomarker of neutrophil extracellular trap formation, predicts the risk of venous thromboembolism in cancer patients." J Thromb Haemost.
Mezouar, S., et al. (2015). "Inhibition of platelet activation prevents the P-selectin and integrin-dependent accumulation of cancer cell microparticles and reduces tumor growth and metastasis in vivo." Int J Cancer. 136(2):462-475.
Thomas, G. M., et al. (2009). "Cancer cell-derived microparticles bearing P-selectin glycoprotein ligand 1 accelerate thrombus formation in vivo." J Exp Med. 206(9):1913-1927.
von Brühl, M. L., et al. (2012). "Monocytes, neutrophils, and platelets cooperate to initiate and propagate venous thrombosis in mice in vivo." J Exp Med. 209(4):819-835.

## Claims

1. A protein or polypeptide comprising a polypeptide that has at least 85 % sequence identity with the amino acid sequence SEQ ID NO: 1 for use for treating and/or preventing thromboinflammation.

2. The protein or polypeptide for use according to claim **1,** wherein said protein or polypeptide comprises a polypeptide having the amino acid sequence SEQ ID NO: 2.

3. The protein or polypeptide for use according to claim **1,** wherein said protein or polypeptide comprises a polypeptide having the amino acid sequence SEQ ID NO: 1.

4. The protein or polypeptide for use according to any one of claims **1** to **3,** wherein said thromboinflammation is selected from the group comprising atherosclerosis, plaque rupture, devices-induced thromboinflammation, thrombosis induced by catheterism and/or stent positioning procedures, thrombosis induced by extracorporeal circulation, thrombus formation following cerebral injuries, coronary artery disease, acute myocardial infraction, cancer-associated thrombosis, metastasis-associated thrombosis, stroke-associated thrombosis, Behçet's disease (BD), antineutrophil cytoplasmic antibody-associated (ANCA) vasculitides, Takayasu arteritis, rheumatoid arthritis, systemic lupus erythematosus, antiphospholipid syndrome, familial Mediterranean fever, thromboangiitis obliterans (TAO), sepsis, inflammatory bowel diseases, heparin-induced thrombocytopenia, immunothrombosis, thrombosis associated with preeclampsia, thrombotic complication in cell and cell cluster transplantation and in whole organ transplantation or grafts, venous thromboembolism, aneurysms, and skeletal muscle ischemia-reperfusion syndrome.

5. The protein or polypeptide for use according to any one of claims **1** to **3,** wherein said thromboinflammation is atherosclerosis.

6. The protein or polypeptide for use according to any one of claims **1** to **3,** wherein said thromboinflammation is thrombosis induced by catheterism and/or stent positioning procedures.

7. The protein or polypeptide for use according to any one of claims **1** to **3,** wherein said thromboinflammation is blood contacting medical devices-induced thromboinflammation.

8. The protein or polypeptide for use according to any one of claims **1** to **3,** wherein said thromboinflammation is plaque rupture.

9. The protein or polypeptide for use according to any one of claims **1** to **3,** wherein said thromboinflammation is thrombosis associated with the extracorporeal circulation.

10. A pharmaceutical composition comprising a protein or polypeptide comprising a polypeptide that has at least 85 % sequence identity with the amino acid sequence SEQ ID NO: 1 and at least one pharmaceutically acceptable excipient for use according to any one of claims **1** to **9.**

11. A medicament comprising a protein or polypeptide comprising a polypeptide that has at least 85 % sequence identity with the amino acid sequence SEQ ID NO: 1 for use according to any one of claims **1** to **9.**

12. A medical device coated with an isolated polypeptide that has at least 85 % sequence identity with the amino acid sequence SEQ ID NO: 1 for use according to any one of claims **1** to **9.**

13. A kit comprising a protein or polypeptide comprising an isolated polypeptide that has at least 85 % sequence identity with the amino acid sequence SEQ ID NO: 1 for use according to any one of claims **1** to **9.**

## Patentansprüche

1. Protein oder Polypeptid, umfassend ein Polypeptid, das mindestens 85 % Sequenzidentität mit der Aminosäuresequenz SEQ ID Nr. 1 aufweist, zur Verwendung zur Behandlung und/oder Prävention einer Thromboinflammation.

2. Protein oder Polypeptid zur Verwendung nach Anspruch **1,** wobei das Protein oder Polypeptid ein Polypeptid mit der Aminosäuresequenz SEQ ID Nr. 2 umfasst.

3. Protein oder Polypeptid zur Verwendung nach Anspruch **1,** wobei das Protein oder Polypeptid ein Polypeptid mit der Aminosäuresequenz SEQ ID Nr. 1 umfasst.

4. Protein oder Polypeptid zur Verwendung nach einem der Ansprüche **1** bis **3,** wobei die Thromboinflammation aus der Gruppe umfassend Atherosklerose, Plaqueruptur, durch Vorrichtungen induzierte Thromboinflammation, durch Katheterismus und/oder Stentpositionierungsvorgänge induzierte Thrombose, durch den extrakorporalen Kreislauf induzierte Thrombose, Thrombusbildung nach Hirnverletzungen, koronare Herzkrankheit, akuten Myokardinfarkt, mit Krebs assoziierte Thrombose, mit einer Metastase assoziierte Thrombose, mit einem Schlaganfall assoziierte Thrombose, Morbus Adamantiades-Behçet (ABD), mit antineutrophilen zytoplasmatischen Antikörpern assoziierten (ANCA) Vaskulitiden, Takayasu-Arteriitis, rheumatoide Arthritis, systemischen Lupus erythematodes, Antiphospholipidsyndrom, familiäres Mittelmeerfieber, Thromboangiitis obliterans (TAO), Sepsis, chronisch-entzündliche Darmerkrankungen, durch Heparin induzierte Thrombozytopenie, Immunothrombose, mit Präeklampsie assoziierte Thrombose, thrombotische Komplikation bei Zell- und Zellhaufentransplantation und bei Vollorgantransplantation oder Transplantaten, venöse Thromboembolie, Aneurysmen und Skelettmuskel-Ischämie-Reperfusionssyndrom ausgewählt ist.

5. Protein oder Polypeptid zur Verwendung nach einem der Ansprüche **1** bis **3,** wobei die Thromboinflammation eine Atherosklerose ist.

6. Protein oder Polypeptid zur Verwendung nach einem der Ansprüche **1** bis **3,** wobei die Thromboinflammation eine durch Katheterismus und/oder Stentpositionierungsvorgänge induzierte Thrombose ist.

7. Protein oder Polypeptid zur Verwendung nach einem der Ansprüche **1** bis **3,** wobei die Thromboinflammation eine durch mit Blut in Kontakt kommende medizinische Vorrichtungen induzierte Thromboinflammation ist.

8. Protein oder Polypeptid zur Verwendung nach einem der Ansprüche **1** bis **3,** wobei die Thromboinflammation eine Plaqueruptur ist.

9. Protein oder Polypeptid zur Verwendung nach einem der Ansprüche **1** bis **3,** wobei die Thromboinflammation eine mit dem extrakorporalen Kreislauf assoziierte Thrombose ist.

10. Pharmazeutische Zusammensetzung, umfassend ein Protein oder Polypeptid, umfassend ein Polypeptid, das mindestens 85 % Sequenzidentität mit der Aminosäuresequenz SEQ ID Nr. 1 aufweist, und mindestens einen pharmazeutisch unbedenklichen Hilfsstoff, zur Verwendung nach einem der Ansprüche **1** bis **9.**

11. Arzneimittel, umfassend ein Protein oder Polypeptid, umfassend ein Polypeptid, das mindestens 85 % Sequenzidentität mit der Aminosäuresequenz SEQ ID Nr. 1 aufweist, zur Verwendung nach einem der Ansprüche **1** bis **9.**

12. Medizinische Vorrichtung, die mit einem isolierten Polypeptid beschichtet ist, das mindestens 85 % Sequenzidentität mit der Aminosäuresequenz SEQ ID Nr. 1 aufweist, zur Verwendung nach einem der Ansprüche **1** bis **9.**

13. Kit, umfassend ein Protein oder Polypeptid, umfassend ein isoliertes Polypeptid, das mindestens 85 % Sequenzidentität mit der Aminosäuresequenz SEQ ID Nr. 1 aufweist, zur Verwendung nach einem der Ansprüche **1** bis **9.**

## Revendications

1. Protéine ou polypeptide comprenant un polypeptide ayant au moins 85 % d'identité de séquence avec la séquence d'acides aminés SEQ ID NO : 1, pour son utilisation pour traiter et/ou prévenir la thromboinflammation.

2. La protéine ou le polypeptide pour son utilisation selon la revendication **1,** dans laquelle ladite protéine ou ledit polypeptide comprend un polypeptide ayant la séquence d'acides aminés SEQ ID NO : 2.

3. La protéine ou le polypeptide pour son utilisation selon la revendication **1,** dans laquelle ladite protéine ou ledit polypeptide comprend un polypeptide ayant la séquence d'acides aminés SEQ ID NO : 1.

4. La protéine ou le polypeptide pour son utilisation selon l'une quelconque des revendications **1** à **3,** dans laquelle ladite thromboinflammation est choisie dans le groupe comprenant l'athérosclérose, la rupture de plaque, la thromboinflammation induite par des dispositifs, la thrombose induite par des procédures de cathétérisme et/ou de mise en place d'un stent, thrombose induite par la circulation extracorporelle, formation de thrombus à la suite de lésions cérébrales, maladie coronarienne, infarctus aigu du myocarde, thrombose associée au cancer, thrombose associée aux métastases, thrombose associée aux accidents vasculaires cérébraux, maladie de Behçet (BD), vascularites associées aux anticorps antineutrophiles cytoplasmiques (ANCA), artérite de Takayasu, polyarthrite rhumatoïde, lupus érythémateux disséminé, syndrome des antiphospholipides, fièvre méditerranéenne familiale, thromboangéite oblitérante (TAO), septicémie, maladies inflammatoires de l'intestin, thrombocytopénie induite par l'héparine, immunothrombose, thrombose associée à la pré-éclampsie, complication thrombotique dans la transplantation de cellules et de groupes de cellules et dans la transplantation ou la greffe d'organes entiers, thromboembolie veineuse, anévrismes, et syndrome d'ischémie-reperfusion des muscles squelettiques.

5. La protéine ou le polypeptide pour son utilisation selon l'une quelconque des revendications **1** à **3,** dans laquelle la thromboinflammation est l'athérosclérose.

6. La protéine ou le polypeptide pour son utilisation selon l'une quelconque des revendications **1** à **3,** dans laquelle ladite thromboinflammation est une thrombose induite par des procédures de cathétérisme et/ou de mise en place d'un stent.

7. La protéine ou le polypeptide pour son utilisation selon l'une quelconque des revendications **1** à **3,** dans laquelle ladite thromboinflammation est une thromboinflammation induite par des dispositifs médicaux en contact avec le sang.

8. La protéine ou le polypeptide pour son utilisation selon l'une quelconque des revendications **1** à **3,** dans laquelle la thromboinflammation est une rupture de plaque.

9. La protéine ou le polypeptide pour son utilisation selon l'une quelconque des revendications **1** à **3,** dans laquelle la thromboinflammation est une thrombose associée à la circulation extracorporelle.

10. Composition pharmaceutique comprenant une protéine ou un polypeptide comprenant un polypeptide ayant au moins 85 % d'identité de séquence avec la séquence d'acides aminés SEQ ID NO : 1 et au moins un excipient pharmaceutiquement acceptable, pour son utilisation selon l'une quelconque des revendications **1** à **9.**

11. Médicament comprenant une protéine ou un polypeptide comprenant un polypeptide ayant au moins 85 % d'identité de séquence avec la séquence d'acides aminés SEQ ID NO : 1, pour son utilisation selon l'une quelconque des revendications **1** à **9.**

12. Dispositif médical recouvert d'un polypeptide isolé ayant au moins 85 % d'identité de séquence avec la séquence d'acides aminés SEQ ID NO : 1, pour son utilisation selon l'une quelconque des revendications **1** à **9.**

13. Kit comprenant une protéine ou un polypeptide comprenant un polypeptide isolé ayant au moins 85 % d'identité de séquence avec la séquence d'acides aminés SEQ ID NO : 1, pour son utilisation selon l'une quelconque des revendications **1** à **9.**
